# EUROPÄISCHE PATENTANMELDUNG

(11) **EP 3 106 470 A1**
(43) Veröffentlichungstag der Anmeldung: **21.12.2016**
(21) Anmeldenummer: 15001798.6
(22) Anmeldetag: 17.06.2015
(51) Int. Cl.: C08F 2/04, A61K 8/44, A61K 8/46, A61Q 5/02, A61Q 19/00, A61K 8/81, C08F 220/56, C08F 220/58, C08L 33/24, C08L 33/26

(54) **VERFAHREN ZUR HERSTELLUNG VON POLYMEREN AUF BASIS VON ACRYLOYLDIMETHYLTAURAT UND SPEZIELLEN VERNETZERN**

(71) Anmelder: Clariant International Ltd, 4132 Muttenz (CH)
(72) Erfinder: MUECKENHEIM, Wiebke, 65812 Bad Soden (DE); FISCHER, Dirk, 55270 Klein-Winternheim (DE); BERZ, Katharina, 63500 Seligenstadt (DE); STARKULLA, Gundula, 55130 Mainz (DE)
(74) Vertreter: Mikulecky, Klaus

(57) **Zusammenfassung**

Gegenstand der Erfindung ist ein Verfahren zur Herstellung von wasserlöslichen oder wasserquellbaren Polymeren, ein wasserlösliches oder wasserquellbares Polymer erhältlich nach dem Verfahren und eine kosmetische, dermatologische oder pharmazeutische Formulierung umfassend wasserlöslichen oder wasserquellbaren Polymeren. In einer bevorzugten Ausführungsform vorliegen bis zu 99,9 Mol-% der freien Säuregruppe der widerkehrenden Struktureinheiten des Polymers hervorgegangen aus Acryloyldimethyltaurat als neutralisiertes Alkali-Salz oder Erdalkali-Salz, vorzugsweise als Natrium-Salz.

## Beschreibung

Die vorliegende Erfindung betrifft ein Verfahren zur Herstellung von wasserlösliche oder wasserquellbare Polymere auf Basis von Acryl-, Methacryl- oder Ethacrylamidoalkylsulfonsäure oder deren salzen und speziellen Vernetzern und die Verwendung dieser Polymere in kosmetischen, pharmazeutischen Formulierungen.

In der Industrie werden regelmäßig synthetische Polymere auf Basis des Monomers Acryloyldimethyltaurat in verschiedensten Anwendungen eingesetzt. Die Kosmetikindustrie verwendet Polymere auf Basis von Acryloyldimethyltaurat als Rheologie-modifizierer beispielsweise in Cremes, Emulsionen und Hydrogelen. WO0139449, und EP0815844 beschrieben kosmetische Formulierungen auf Basis von Öl in Wasser Emulsionen, die Co-Polymere von Acryloyldimethyltaurat enthalten. In EP1570836 werden wässrige Gele beschrieben, die Co-Polymere von Acryloyldimethyltaurat enthalten und als kosmetische Selbstbräuner Verwendung finden.

EP2579946 beschriebt eine Formulierung für Dekorative Kosmetik, die neben Poly(acryloyldimethyltaurat) Copolymeren farbige Pigment Partikel enthält.

Die Patente WO0243689, WO0243687, US8062630, EP1779895, US7338534, EP1203789, EP1166770, EP0850642, EP0815847, EP0815845 und EP0815828 beschreiben weitere kosmetische Formulierungen für die Haut-, Haar-, und Körperpflege. Auch in diesen kosmetischen Formulierungen finden Co-Polymere auf Basis des Monomers Acryloyldimethyltaurat Verwendung.

EP1477553 beschriebt die Verwendung von der genannten Polymere in flüssigen Wasch und Reinigungsformulierungen. Dort werden vernetzte Copolymeren des Poly(acryloyldimethyltaurats) zur Erhöhung der Viskosität in der Formulierung eingesetzt.

Neben kosmetischen und pharmazeutischen Formulierungen werden Copolymere auf Basis des Monomers Acryloyldimethyltaurat in vielen weiteren Anwendungen eingesetzt.

Die synthetischeren Poly(acryloyldimethyltaurat) Copolymere können bei der industriellen Herstellung in zwei verschiedenen physikalischen Formen anfallen, als Pulver und in flüssiger Form. Unter der flüssigen Form sind Polymerlösungen zu verstehen wie beispielsweise Polymeremulsionen oder Dispersionen in denen das Polymer in einem Lösemittel gelöst oder durch den Einsatz eines Emulgator dispergiert vorliegt.

Poly(acryloyldimethyltaurat) Copolymere in Pulverform wurden kürzlich in den Patentanmeldungen, US5373044, US2798053, EP301532, EP 816403, EP1116733 und EP 1069142 beschrieben. Alle diese Polymere auf Basis von Acryloyldimethyltaurat werden mit Hilfe einer Fällungspolymerisation erhalten. Hierbei werden die verwendeten Mono-, und Comonomere in einem organischen Lösemittel, wie Toluol, Ethylacetat, Hexan, Cyclohexan, Ethanol oder 2-Methylpropan-2-ol hergestellt. Der Nachteil dieser organischen Lösemittel ist meist, das sich das Acryloyldimethyltaurat nicht vollständig löst und es nach der Polymerisation zu hohen Restmonomer Anteilen erhaltenen Polymer kommt. Zudem werden meist keine hohen Molaren Massen erreicht, da das Polymer während der Polymerisation im Lösemittel zu schnell unlöslich wird. Poly(acryloyldimethyltaurat) Copolymere die mit Hilfe einer Fällungspolymerisation hergestellt wurden, haben im Vergleich zu Inversenemulsions-, oder Emulsionspolymerisation den Vorteil, dass im finalen Produkt keine Reste von Öl und den Emulgatoren vorliegen. Die eingesetzten Öle und die eingesetzten Emulgatoren in den genannten Polymerisationsverfahren können zum Teil Hautirritationen hervorrufen. Zudem haben die Polymere, die mit Hilfe einer Inversenemulsions-polymerisation hergestellt wurden meist den Nachteil, dass das im Polymer enthalten Öl aus dem Verfahren zu Eintrübungen in wässrigen Polymerlösungen führt.

In WO2010108634, WO2012119747, WO2012119746, EP0816403, EP2227498, US7151137 und WO0244268 werden unteranderem Verfahren zu Herstellung von Poly(acryloyldimethyltaurat) Copolymeren die mit Hilfe einer Fällungspolymerisation in 2-Methylpropan-2-ol beschrieben.

Die Verwendung von 2-Methylpropan-2-ol oder 2-Methylpropan-2-ol/Wasser Gemische macht es notwendig das Acryloyldimethyltaurat mit gasförmigen Ammoniak oder einem Ammonium haltigen Salz zu neutralisieren, da dies die einzigen Salze des Acryloyldimethyltaurats in 2-Methylpropan-2-ol nur bedingt löslich sind. Die geringe Löslichkeit dieser Salze beeinflusst auf negativer Weise das Molarekolaurgewicht der finalen Polymere. Dieses wiederum wirkt sich negative auf die Viskosität der wässrigen Gele aus. Für Anwendungen, in denen diese Polymere als Rheologiemodifizierer eingesetzt werden, ist es wichtig, dass die wässrigen Lösungen dieser Polymere auch bei geringen Polymerkonzentrationen hohe Viskositäten aufbauen.

In den Vergleichbesipielen A1/A2, wurden das Ammoniumsalz und das korrespondierende Natriumsalz unter Verwendung von Natriumhydrogencarbonat nach EP0816403 hergestellt. Der negative Einfuss der Löslichkeit der Salze des Acryloyldimethyltaurate während der Polymerisation auf die Viskosität wässrigen Lösungen der finalen Polymere kann eindeutig demonstriert werden. Während im Vergleichspolymer A1 (NH₄⁺-Salz) 1 Gew.-% gelöst in Wasser eine Viskosität von 34400 mPa*s gemessen wird, konnte im Vergleichspolymer A2 (Na⁺-Salz) 1 Gew.-% gelöst in Wasser nur eine Viskosität von 92 mPa*s gemessen werden.

In US2012095120 wird ein Verfahren zu Herstellung von Natriumsalzen des Poly(acryloyldimethyltaurat) Copolymeren die mit Hilfe einer Fällungspolymerisation in Ketonen beschrieben. Das Vergleichpolymer B1 hergestellt nach US2012095120 zeigt als 1 Gew.-%wässrige Lösung zwar mit 16000 mPa*s eine deutlich höhere Viskosität als das Vergleichspolymer A2, liegt aber immer noch deutlich unter der Viskosität (40000 mPa*s) eines vergleichbaren erfindungsgemäßen Polymers. Die wässrigen Lösungen des Vergleichpolymers B1 sind sehr trübe und weisen unlösliche Anteile auf. Dies ist vorallen in kosmetischen, pharmazeutischen Hydrogel-Formulierungen ein Nachteil, da dort klare Gele vom Verbraucher gefordert werden.

Ammonium salze der Poly(acryloyldimethyltaurat) Copolymere haben vor allem in Formulierungen mit einen pH-Wert > 7 den entscheidenden Nachteil, dass es zur Freisetzung von Ammoniakgas kommt, was ein spezielles technisches Equipment erforderlich macht, um beispielsweise eine Personengefährdung auszuschließen. Freie Ammonium Ionen können zudem zu unerwünschten Nebenreaktionen führen. Dies ist beispielsweise bei Dihydroxyaceton (DHA), Ascorbinsäure, Ascorbylglucosid, Natriumascorbylphosphat, der Fall, wo es zu unerwünschten Farbänderungen in der kosmetischen oder pharmazeutischen Formulierung kommt.

Die Aufgabe der vorliegenden Erfindung war es daher, ein Verfahren zur Herstellung von Polymeren und Co-Polymeren des Acryloyldimethyltaurats bereitzustellen, mit deren Hilfe die Metallsalze, vorzugsweise Alkali- und Erdalkalisalze, dieser Polymere und Co-Polymere direkt darstellbar sind. Diese Polymere und Co-Polymere sollen eine verbesserte Performance in der Anwendung als Additiv in kosmetischen und pharmazeutischen Formulierungen zeigen. Bei ihrer Anwendung in alkalischen Formulierungen kommt es nicht zur Freisetzung von Ammoniak, welches mit anderen Additiven wie Dihydroxyaceton (DHA), Ascorbinsäure, Ascorbylglucosid, Natriumascorbylphosphat zu unerwünschten Nebenreaktionen wie Verfärbungen führt. In ihrer Eigenschaft alsRheologiemodifizierer zeigen die Alkali- und Erdalkalisalze dieser Polymere und Co-Polymere eine deutlich hörere Viskosität wie es für Rheologiemodifizierer des Standes der Technik üblich war. Überraschenderweise wurde nun gefunden, dass lineare, verzweigte oder vernetzte Polymere oder Co-Polymere des Acryloyldimethyltaurats, die als Metallsalze, vorzugsweise Alkali- oder Erdalkalisalze, frei von Ammoniumsalzen sind, mit Hilfe eines Verfahrens hergestellt werden können, indem das Acryloyldimethyltaurat als neutralisiertes Metallsalz, vorzugsweise Alkali-Salz oder Erdalkali-Salz, insbesondere vorzugsweise als NatriumSalz polymerisiert wird.

Gegenstand der vorliegenden Erfindung ist ein Verfahren zur Herstellung von wasserlösliche oder wasserquellbare Polymere enthaltend:
a) 95 bis 99,99 Mol-% vorzugsweise 97 bis 99,9 Mol-% einer oder mehrerer wiederkehrender Struktureinheiten der Formel (1) 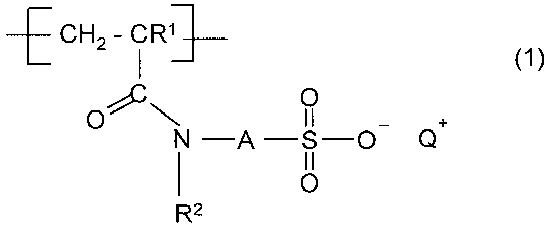 worin
   - R¹, R²: Wasserstoff, Methyl oder Ethyl,
   - A: lineares oder verzweigtes C₁-C₁₂-Alkylen, und
   - Q⁺: für H⁺, Li⁺, Na⁺, K⁺, ½ Ca⁺⁺, ½ Mg⁺⁺, ½ Zn⁺⁺, 1/3 Al⁺⁺⁺- oder für Mischungen aus diesen Ionen steht, vorzugsweise für Na⁺steht,
b) 0,01 bis 5, vorzugsweise 0,01 bis 3 Mol-% an einer oder mehreren voneinander unabhängigen wiederkehrenden vernetzenden Struktureinheiten, die aus einem oder mehreren Monomeren mit mindestens zwei olefinischen Doppelbindungen hervorgegangen sind,
und dadurch gekennzeichnet ist, dass Monomere, aus denen sich die Struktureinheiten der Komponenten a) und b) ableiten, in einem polaren Lösungsmittel oder Lösungsmittelgemisch, in Fällung radikalisch polymerisiert werden, wobei in dem Fall das polare Lösungsmittel oder Lösungsmittelgemisch 2-Methyl-2-Propanol oder ein Keton enthält, ein zweites polares organisches Lösungsmittel enthalten sein muss, und gegebenenfalls die Monomere vor der Polymerisation oder das Polymer nach der Polymerisation mit einer Li⁺-, Na⁺-, K⁺-, Ca⁺⁺-, Mg⁺⁺-, Zn⁺⁺- oder Al⁺⁺⁺-enthaltenden Base vorzugsweise mit den entsprechenden Hydroxiden, Hydrogencarbonaten und Carbonaten, neutralisiert werden.

Die nach dem erfindungsgemäßen Verfahren hergestellten Polymere, werden im Folgenden als "Polymer A", oder als "Polymere A" bezeichnet.

Die Polymere A können jeweils verschiedene Struktureinheiten der Formel (1) oder der Komponente B enthalten sein. Ein Polymer A kann beispielsweise mehrere Struktureinheiten der Formel (1) enthalten, die sich durch unterschiedliche Gegenionen Q+ voneinander unterscheiden. Ein weiteres Polymer A kann beispielsweise auch mehrere Struktureinheiten der Komponente B enthalten, die sich durch unterschiedliche Anzahl der olefinischen Doppelbindungen unterscheiden.

Bevorzugt ist die eine oder sind die mehreren Struktureinheiten der Formel (1) der Polymere A abgeleitet von Monomeren aus der Gruppe bestehend aus Acryloyldimethyltaurat, Acryloyl-1,1-dimethyl-2methyltaurat, Acryloyltaurat, Acryloyl-N-methyltaurat vorzugsweise Acryloyldimethyltaurat.

Vorzugsweise ist der Neutralisationsgrad der einen oder der mehreren Struktureinheiten der Formel (1) der Polymere A von 50,0 bis 100 Mol-%, besonders bevorzugt von 80,0 bis 100 Mol-%, insbesondere bevorzugt von 90,0 bis 100 Mol-% und außerordentlich bevorzugt von 95,0 bis 100 Mol-%.

In der einen oder den mehreren Struktureinheiten der Formel (1) der Polymere A ist das von H⁺ verschiedene Gegenion Q⁺ vorzugsweise ausgewählt Alkali⁺, wobei unter Alkali⁺ wiederum Na⁺ bevorzugt ist, Erdalkali⁺⁺ und Mischungen aus diesen Ionen. Besonders bevorzugt ist das von H⁺ verschiedene Gegenion Q⁺ Na⁺.

Bevorzugte Polymere A enthalten 96 bis 99,7 Mol-%, insbesondere 97 bis 99,5 Mol-% an Struktureinheiten der allgemeinen Formel (1), vorzugsweise abgeleitet von NatriumSalz des Acryloyldimethyltaurats und 0,3 bis 4 Mol-% insbesondere 0,5 bis 3 Mol-% an einer oder mehreren voneinander unabhängigen wiederkehrenden vernetzenden Struktureinheiten, die aus einem oder mehreren Monomeren mit mindestens zwei olefinischen Doppelbindungen hervorgegangen sind.

Die eine oder die mehreren vernetzenden Struktureinheiten der Komponente b) der Polymere A leiten sich vorzugsweise ab von Acryl- oder Methacrylsäureestern, Acryl- oder Methacrylsäureamiden, Polyglykoldiacryl- oder -methacrylsäureestern, Polyglykoldiacryl- oder methacrylsäureamiden, Dipropylenglykoldiacryl- oder methacrylsäureestern, Dipropylenglykoldiacryl- oder methacrylsäureamiden, ethoxylierten Glycerindiacrylaten oder -methacrylaten, ethoxylierten Glycerintriacrylaten oder -methacrylaten, propoxylierten Glycerindiacrylaten oder -methacrylaten, propoxylierten Glycerintriacrylaten oder methacrylaten, oder anderen Acryl- oder Methacrylsäureestern, Acryl- oder Methacrylsäureamiden multifunktioneller Alkohole, Trimethylolpropantriacrylaten oder trimethacrylaten, Allyl- oder Vinylethern multifunktioneller Alkohole, Methylen-bis-acrylamid oder Divinylbenzol.

Weiterhin bevorzugt leiten sich die eine oder die mehreren vernetzenden Struktureinheiten der Komponente b) der Polymere A von Monomeren der allgemeinen Formel (2) ab 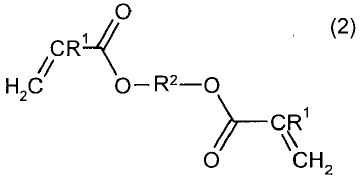 worin
- R¹: Wasserstoff, Methyl oder Ethyl bedeutet und
- R²: eine lineare oder verzweigte Alkylengruppe mit 1 bis 6 Kohlenstoffatomen oder eine lineare oder verzweigte, einfach oder mehrfach ungesättigte Alkenylengruppe mit 2 bis 6 Kohlenstoffatomen ist.

Weiterhin bevorzugt enthalten die Polymere A ein oder mehrere voneinander unabhängige wiederkehrende vernetzende Struktureinheiten der Formel (3) 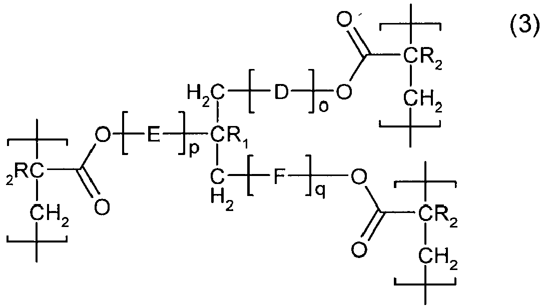 gemäß Komponente b), worin
- R₁: eine lineare oder verzweigte Alkylengruppe mit 1 bis 6 Kohlenstoffatomen bedeutet und
- R₂: jeweils unabhängig voneinander Wasserstoff, Methyl oder Ethyl bedeuten,
- D, E und F: jeweils unabhängig voneinander Methylenoxy (-CH₂-O- oder -O-CH₂-), Ethylenoxy (CH₂-CH₂-O- oder -O-CH₂-CH₂-), Popylenoxy (-CH(CH₃)-CH₂-O oder -O-CH₂-CH(CH₃)-), eine lineare oder verzweigte Alkylengruppe mit 1 bis 6 Kohlenstoffatomen, eine lineare oder verzweigte, einfach oder mehrfach ungesättigte Alkenylengruppe mit 2 bis 6 Kohlenstoffatomen, eine lineare Mono-Hydroxyalkylengruppe mit 2 bis 6 Kohlenstoffatomen oder eine lineare oder verzweigte Di-Hydroxyalkylengruppe mit 3 bis 6 Kohlenstoffatomen bedeuten, und
- o, p und q: jeweils unabhängig voneinander eine ganze Zahl von 0 bis 50, vorzugsweise 0 bis 25 und insbesondere bevorzugt von 0 bis 10 bedeutet.

Besonders bevorzugt als Vernetzer für die Polymere A sind Glycerinpropoxylattriacrylat (GPTA), Trimethylolpropantriacrylat (TMPTA), Pentaerythritoldiacrylatmonostearat (PEAS), Hexandioldiacrylat (HDDA) und Hexandioldimethacrylat (HDDMA). Insbesondere bevorzugt ist Glycerinpropoxylattriacrylat (GPTA) und Trimethylolpropantriacrylat (TMPTA)

Die Verteilung der verschiedenen Struktureinheiten in den Polymeren A kann statistisch, blockartig, alternierend oder Gradienten artig sein.

Die Herstellung der Polymere A erfolgt mittels radikalischer Fällungspolymerisation in einem polaren Lösungsmittel oder Lösungsmittelgemisch. Hierbei werden die entsprechenden Monomere aus denen sich die Struktureinheiten der Komponenten a) und b) ableiten in einem polaren Lösungsmittel oder Lösungsmittelgemisch gelöst oder dispergiert und die Polymerisation in an sich bekannter Weise, z. B. durch Zugabe einer radikalbildenden Verbindung, gestartet. Dabei können beispielsweise die vorgelegten Monomere "direkt" polymerisiert werden. Sie können aber auch vor der Polymerisation neutralisiert werden, indem beispielsweise saure Gruppen eingesetzter Monomere vor der Polymerisation mit Basen ungesetzt werden, wobei die Gegenionen Q⁺ der Struktureinheiten gemäß Formel (1) ausgebildet werden. Anstelle der Neutralisation der Monomere vor der Polymerisation können jedoch auch die Polymere nach der erfolgten Polymerisation mit den Basen neutralisiert werden.

In einer weiteren bevorzugten Ausführungsform des erfindungsgemäßen Verfahrens zur Herstellung der Polymere A, werden die Monomere, aus denen sich die Struktureinheiten der Komponenten a) und b) ableiten, in einem polaren Lösungsmittel oder Lösungsmittelgemischen, radikalisch polymerisiert, und gegebenenfalls die Monomere vor der Polymerisation oder das Polymer A nach der Polymerisation mit einer Li⁺-, Na⁺-, K⁺-, Ca⁺⁺-, Mg⁺⁺-, oder Zn⁺⁺- enthaltenden Base, vorzugsweise mit den entsprechenden Hydroxiden, Hydrogencarbonaten und Carbonaten und besonders bevorzugt mit Hydrogencarbonaten und Carbonaten, neutralisiert.

Bevorzuge Basen zur Neutralisation Struktureinheiten der Komponenten a) sind Natriumhydrogencarbonat, Natriumcarbonat, Natriumhydroxid, Kaliumhydrogencarbonat, Kaliumcarbonat, Kaliumhydroxid, Lithiumhydrogencarbonat, Lithiumcarbonat, Lithiumhydroxid, vorzugsweise Natriumhydrogencarbonat, Natriumcarbonat, Natriumhydroxid, Kaliumhydrogencarbonat, Kaliumcarbonat, Kaliumhydroxid, besonders bevorzugt sind Natriumhydrogencarbonat, Natriumcarbonat, Natriumhydroxid, und insbesondere bevorzugt sind Natriumhydrogencarbonat und Natriumcarbonat.

In einer weiteren bevorzugten Ausführungsform des erfindungsgemäßen Verfahrens zur Herstellung der Polymere A erfolgt die radikalischer Fällungspolymerisation in einem polaren Lösungsmittel oder Lösungsmittelgemisch, dass dadurch gekennzeichnet ist, dass das Lösungsmittel oder Lösungsmittelgemisch einen Siedepunkt von 60 bis 110 °C, vorzugsweise von 60 bis 95 °C, besonders bevorzugt von 65 bis 90 °C aufweist.

In einer weiteren bevorzugten Ausführungsform des erfindungsgemäßen Verfahrens zur Herstellung der Polymere A enthält das polare Lösungsmittel eine Mischung aus:
c) Wasser
   und
d) einem oder mehreren weiteren polare Lösungsmitteln

In einer weiteren bevorzugten Ausführungsform des erfindungsgemäßen Verfahrens besteht die Komponente d) aus einem Lösungsmittelgemisch, welches ein oder mehr polare organische Lösungsmittel enthält.

In einer besonders bevorzugten Ausführungsform des erfindungsgemäßen Verfahrens besteht die Komponente d) aus einem Lösungsmittelgemisch, welches ein oder mehrerer Alkohole und ein oder mehrerer Ketone enthält.

In einer weiteren bevorzugten Ausführungsform des erfindungsgemäßen Verfahrens enthält die Komponente d) ein oder mehrere polare Lösungsmittel ausgewählt aus der Gruppe aus Methanol, Ethanol, 1-Propanol, 2-Propanol, 2-Methyl-2-Propanol, 1-Butanol, 2-Butanol, Dimethylketon, Diethylketon, Pentan-2-on, Butanon, Tetrahydropyran, Tetrahydrofuran, 2-Methyltetrahydrofuran, 1,3-Dioxan, 1,4-Dioxan vorzugsweise, Ethanol, 1-Propanol, 2-Propanol, 2-Methylpropan-2-ol, 1-Butanol, 2-Butanol, Dimethylketon, Tetrahydrofuran, 2-Methyltetrahydrofuran, 1,3-Dioxan, besonders bevorzugt 2-Propanol, 2-Methylpropan-2-ol, Dimethylketon, Tetrahydrofuran, 2-Methyltetrahydrofuran, 1,3-Dioxan ,insbesondere bevorzugt 2-Methylpropan-2-ol und Dimethylketon.

Im erfindungsgemäßen Verfahren können innerhalb der Komponente d) verschiedene polare organische Losemittel enthalten sein. Ein erfindungsgemäßes polares Lösungsmittel der der Komponente d) kann beispielsweise Dimethylketon enthalten. Ein weiteres erfindungsgemäßes polares Lösungsmittel der der Komponente d) kann beispielsweise eine Mischung aus 2-Methylpropan-2-ol und Dimethylketon enthalten. Ein weiteres erfindungsgemäßes Lösungsmittel der Komponente d) kann beispielsweise eine Mischung aus Dimethylketon, 2-Methylpropan-2-ol und Tetrahydrofuran enthalten.

Eine besondere Ausführungsform des erfindungsgemäßen Verfahrens ist dadurch gekennzeichnet, dass das polare Lösungsmittelgemisch 0,5 bis 10 Gew.-%, vorzugsweise 1 bis 8 Gew.-% Wasser und besonders bevorzugt 2 bis 5 Gew.-% Wasser enthält.

Eine weitere besondere Ausführungsform des erfindungsgemäßen Verfahrens ist dadurch gekennzeichnet, dass das polare Lösungsmittelgemisch 1 bis 99,5 Gew.-% vorzugsweise 5 bis 95 Gew.-% und besonders bevorzugt 10 bis 90 Gew.-% 2-Methylpropan-2-ol enthält.

Eine weitere besondere Ausführungsform des erfindungsgemäßen Verfahrens ist dadurch gekennzeichnet, dass das polare Lösungsmittelgemisch aus 0,5 bis 10 Gew.-% Wasser, 1 bis 98,5 Gew.-% 2-Methylpropan-2-ol und 1 bis 98,5 Gew.-% Dimethylketon, vorzugsweise 0,5 bis 7,5 Gew.-% Wasser, 5 bis 94,5 Gew.-% 2-Methylpropan-2-ol und 5 bis 94,5 Gew.-% Dimethylketon, besonders bevorzugt 1 bis 5 Gew.-% Wasser, 7,5 bis 91,5 Gew.-% 2-Methylpropan-2-ol und 7,5 bis 91,5 Gew.-% Dimethylketon enthält.

Eine besonders bevorzugte Ausführungsform des erfindungsgemäßen Verfahrens erfolgt vorzugsweise in einem Gemisch aus 2-Methylpropan-2-ol, Dimethylketon und Wasser. Der Wassergehalt dieses Gemisches darf 10 Gew.-% nicht überschreiten, da sonst im Verlauf der Polymerisation Klumpenbildung auftreten kann. Konkret hat die Wahl der Art und der Menge des Lösungsmittelgemisches so zu erfolgen, dass das Salz der wiederkehrenden Struktureinheit der Formel 1, insbesondere des Acryloyldimethyltaurats darin weitgehend löslich oder dispergierbar ist. Unter weitgehend löslich oder dispergierbar ist zu verstehen, dass sich auch nach Abstellen des Rührwerks kein festes Material aus der Lösung oder Dispersion absetzt. Das im Verlauf der Reaktion entstehende Polymer A soll hingeben in dem gewählten Lösungsmittelgemisch weitgehend unlöslich sein. Unter weitgehend unlöslich ist hierbei zu verstehen, dass im Verlauf der Polymerisation eine gut rührbare, breiige Polymerpaste entsteht, in der sich keine Klumpen oder Verklebungen bilden dürfen. Das durch Absaugen der Paste erhältliche Filtrat darf einen Feststoffgehalt von maximal 5 Gew.-% aufweisen. Sind die Copolymere in stärkerem Ausmaß im gewählten Lösungsmittel oder Lösungsmittelgemisch löslich, kann es beim Trocknen der Polymerisatpaste zu Verklumpungen kommen.

Die Polymerisationsreaktion selbst wird in an sich bekannter Weise durch radikalbildende Verbindungen wie Azoinitiatoren (z.B. Azo-bis-isobutyronitril, 2,2'-Azobis(4-methoxy-2.4-dimethyl valeronitril), 2,2'-Azobis(2.4-dimethyl valeronitril), Dimethyl 2,2'-azobis(2-methylpropionat), 2,2'-Azobis(2-methylbutyronitril), 1,1'-Azobis(cyclohexane-1-carbonitril) oder 2,2'-Azobis[N-(2-propenyl)-2-methyl-propionamid]), Peroxiden (z.B. Dilaurylperoxid, tert.-Butylhydroperoxid, Di-tert.-Butylperoxid, Triphenylmethylhydroperoxid, Benzoylperoxid), oder Persulfaten in einem geeigneten Temperaturintervall von 20 bis 120°C, vorzugsweise zwischen 30 und 80°C und insbesondere bevorzugt zwischen 40 und 70°C, ausgelöst und über einen Zeitraum von 30 min. bis mehreren Stunden fortgeführt.

Die Polymere A fallen als weißer, voluminöser Niederschlag im polaren Lösungsmittelgemisch an. Zur Isolierung können alle üblichen Verdampfungs-, Trocknungs Isolierprozesse verwendet werden. Insbesondere kann das polare Lösungsmittelgemisch durch eine Druckfiltration oder Destillation vom Produkt abgetrennt werden. Ein geringer Rückstand des polaren Lösungsmittelgemisches ist weder aus sicherheitstechnischen noch aus anwendungstechnischen Gründen bedenklich.

Verwendung in kosmetischen, dermatologischen oder pharmazeutischen Zusammensetzungen:
Die nach dem erfindungsgemäßen Verfahren hergestellten Polymere A eignen sich vorteilhafter Weise zur Herstellung von kosmetischen, dermatologischen oder pharmazeutischen Zusammensetzungen. Diese Formulierungen oder Zusammensetzungen werden im Folgenden als "Zusammensetzung A" oder als "Zusammensetzungen A" bezeichnet"

Zusammensetzungen A sowie kosmetische, dermatologische oder pharmazeutische Zusammensetzungen A enthaltend ein oder mehrere Polymere A.

Die Polymere A zeichnen sich durch eine gute Hautmilde und ein angenehmes Hautgefühl aus. Die Polymere A sind darüber hinaus säurestabil. Da die Polymere A auch bei sauren pH-Werten verdicken, kann man verdickte kosmetische Produkte vorteilhafterweise auch mit organischen Säuren, wie Benzoesäure, Sorbinsäure, Paramethoxybenzoesäure konservieren, da auch bei den notwendigen niedrigen pH-Werten genügend Verdickerleistung zur Verfügung steht. Mit ihnen können klare Lösungen erhalten werden.

Die Polymere A sind darüber hinaus basenstabil und setzen bei alkalischen pH-Werten kein Ammoniak oder Ammonium-Ionen frei. Unter alkalischen pH-Werten sind pH-Werte ≥ 7 zu verstehen. In verdickten kosmetische, dermatologische oder pharmazeutische Zusammensetzungen eignen sich die Polymere A in vorteilhafterweise inbesondere in Kombination mit anderen Additiven wie Dihydroxyaceton (DHA), Ascorbinsäure, Ascorbylglucosid und Natriumascorbylphosphat, ohne dass es zu unerwünschten Farbänderungen bzw. Nebenreaktionen in der Formulierung kommt.

Die Zusammensetzungen A enthalten, bezogen auf die fertigen Zusammensetzungen A, vorzugsweise 0,01 bis 10,0 Gew.-%, besonders bevorzugt 0,1 bis 5,0 Gew.-% und insbesondere bevorzugt 0,5 bis 2,0 Gew.-% an den Polymeren A.

In einer weiteren bevorzugten Ausführungsform haben die Zusammensetzungen A, wie Cremes, Lotions, wässrige und wässrige alkoholische Formulierungen Viskositäten vorzugsweise im Bereich von 100 bis 50.000 mPa*s, besonders bevorzugt im Bereich von 500 bis 30.000 mPa*s und insbesondere bevorzugt im Bereich von 1000 bis 20.000 mPa*s (25 °C, Brookfield RVT, T-C Spindel bei 5 rpm).

In einer weiteren bevorzugten Ausführungsform liegen die Zusammensetzungen A in Form von Fluids, Gelen, Ölen, Schäumen, Sprays, Lotions oder Cremes vor.

Die Zusammensetzungen A liegen vorzugsweise auf wässriger oder wässrig-alkoholischer Basis oder als Öl-in-Wasser Emulsionen vor.

In einer besonders bevorzugten Ausführungsform liegen die Zusammensetzungen A auf wässriger oder wässrig-alkoholischer Basis oder als Öl-in-Wasser Emulsionen vor, insbesondere bevorzugt als wässrige tensidhaltige, wässrig-alkoholische tensidhaltige Zusammensetzungen A, und enthalten vorzugsweise, bezogen auf das Gesamtgewicht der Zusammensetzungen A,
a) bis zu 98,0 Gew.-%, vorzugsweise 60,0 bis 98,0 Gew.-%, besonders bevorzugt 70,0 bis 94,4 Gew.-%, insbesondere bevorzugt 75,0 bis 94,5 Gew.-% einer Wasserphase oder wässrigen-alkoholischen Phase,
b) bis zu 50,0 Gew.-%, vorzugsweise 1,0 bis 30,0 Gew.-%, besonders bevorzugt 5,0 bis 20,0 Gew.-%, insbesondere bevorzugt 5,0 bis 15,0 Gew.-% eines oder mehrerer Tenside -> auch Emulgatoren,
c) bis zu 10,0 Gew.-%, vorzugsweise 0,01 bis 10,0 Gew.-%, besonders bevorzugt 0,1 bis 5,0 Gew.-%, insbesondere bevorzugt 0,5 bis 2,0 Gew.-% an einem oder mehreren der Polymere A und
d) bis zu 50,0 Gew.-%, vorzugsweise 0,5 bis 38,99 Gew.-%, besonders bevorzugt 0,5 bis 6,0 Gew.-%, insbesondere bevorzugt 1,0 bis 5,0 Gew.-% eines oder mehrerer weiterer Zusatzstoffe, wobei der eine oder die mehreren Zusatzstoffe insbesondere ein oder mehrerer Öle bedeuten, wenn die erfindungsgemäße Zusammensetzung als Öl-in-Wasser Emulsionen vorliegen.

Für die Zusammensetzungen A auf wässrig-alkoholischer oder auch alkoholischer Basis kommen alle ein- oder mehrwertigen Alkohole in Betracht. Bevorzugt werden Alkohole mit 1 bis 4 Kohlenstoffatomen wie Ethanol, Propanol, Isopropanol, 1-Butanol, 2-Butanol, 2-Methylpropan-2-ol oder Glycerin sowie Alkylenglykole, insbesondere Propylen-, Butylen- oder Hexylenglykol, und Mischungen aus den genannten Alkoholen. Weitere bevorzugte Alkohole sind Polyethylenglykole mit einer relativen Molekülmasse unter 2.000. Insbesondere ist ein Einsatz von Polyethylenglykol mit einer relativen Molekülmasse zwischen 200 und 600 und von Polyethylenglykol mit einer relativen Molekülmasse zwischen 400 und 600 bevorzugt.

Die Zusammensetzungen A können ein oder mehrere Öle enthalten.

Die Öle können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Methanol, Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel , Wiesenschaumkraut-, Ricinusöl, Olivenöl, Erdnussöl, Rapsöl und Kokosnussöl, sowie synthetische Triglyceridöle, z. B. das Handelsprodukt Myritol 318 sowie das Handelsprodukt Velsan^{®} CCT (Capryl-Caprinsäure-triglycerid, Clariant). Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈-C₂₂-Alkanolen, z.B. die Handelsprodukte Finsolv^{®}SB (Isostearylbenzoat), Finsotv^{®}TN (C₁₂-C₁₅-Alkylbenzoat) und Finsolv^{®}EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z.B. Di n-octylether (Cetiol^{®} OE), Di-n-nonylether, Di-n-decylether, Di-n-undecylether, Di n dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert. Butyln-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 bis 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der Hydroxypropionsäure, der Tartronsäure, der D Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®} S), Ozokerit, und Ceresin.

Ebenso in Betracht kommen Silikonöle bzw. -wachse, vorzugsweise Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41 M65, SilCare^{®} Silicone 41M70, SilCare^{®} Silicone 41M80 (Clariant) erhältlichen Dimethicone, Stearyldimethylpolysiloxan, C20-C24-Alkyl-dimethylpolysiloxan, C₂₄-C₂₈-Alkyl-dimethylpolysiloxan, aber auch die unter SilCare^{®} Silicone 41M40, SilCare^{®} Silicone 41M50 (Clariant) erhältlichen Methicone, weiterhin Trimethylsiloxysilicate [(CH₂)₃SiO)1/2]x[SiO₂]y, wobei x für eine Zahl von 1 bis 500 und y für eine Zahl von 1 bis 500 steht, Dimethiconole R₃SiO[R₂SiO]xSiR₂OH und HOR₂SiO[R₂SiO]xSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

In einer weiteren bevorzugten Ausführungsform enthalten die Zusammensetzungen A zusätzlich noch als schaumverstärkende Mittel Co Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die Zusammensetzungen A können als weitere Hilfs- und Zusatzstoffe beispielsweise Wachse, Emulgatoren, Co-Emulgatoren, Solubilisatoren, Elektrolyte, Hydroxysäuren, Stabilisatoren, kationische Polymere, Filmbildner, weitere Verdicker, Gelierungsmittel, Überfettungsmittel, Rückfetter, antimikrobielle Wirkstoffe, biogene Wirkstoffe, Adstringentien, deodorierende Stoffe, Sonnenschutzfilter, Antioxidantien, Feuchthaltemittel, Lösungsmittel, Farbmittel, Perlglanzmittel, Duftstoffe, Trübungsmittel und/oder Silikone enthalten.

Die erfindungsgemäßen Zusammensetzungen können Wachse, beispielsweise Paraffinwachse, Mikrowachse und Ozokerite, Bienenwachs und ihre Teilfraktionen sowie der Bienenwachsderivate, Wachse aus der Gruppe der homopolymeren Polyethylene oder Coplymere der α-Olefine, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht: Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen

und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z.B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethytenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether.

Ebenso bevorzugt sind Fettsäureethoxylate, gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders Polyethylenglykol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

Besonders vorteilhafte Coemulgatoren sind Glycerylmonostearat, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol, Laurylmethiconcopolyol.

Die Zusammensetzungen A können einen oder mehrere der Emulgatoren, Co-Emulgatoren oder Solubilisatoren in Mengen von 0,1 bis 20,0 Gew.-%, vorzugsweise 1,0 bis 15,0 Gew.-% und besonders bevorzugt 3,0 bis 10,0 Gew.-%, bezogen auf die fertigen Zusammensetzungen, enthalten.

Als Elektrolyt zum Einsatz können kommen anorganische Salze, bevorzugt Ammonium- oder Metallsalze, besonders bevorzugt von Halogeniden, beispielsweise CaCl₂, MgCl₂, LiCl, KCl und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure. Hierzu zählen auch Aluminiumsalze, bevorzugt Aluminiumchlorohydrat oder Aluminium-Zirkonium-Komplexsalze.

In einer bevorzugten Ausführungsform enthalten die Zusammensetzungen A daher ein oder mehrere Substanzen ausgewählt aus anorganischen und organischen Salzen.

Als Elektrolyt können die Zusammensetzungen A auch Mischungen verschiedener Salze enthalten. Der Gehalt an dem einen oder den mehreren Elektrolyten, bezogen auf die gesamte Zusammensetzung A ist vorzugsweise von 0,01 bis 20,0 Gew.-%, besonders bevorzugt von 0,1 bis 10,0 Gew.-% und insbesondere bevorzugt von 0,5 bis 5,0 Gew.-%.

Die Polymere A sind säurestabil und eignen sich vorzugsweise zur Verwendung in kosmetischen, pharmazeutischen und/oder dermatologischen Zusammensetzungen A mit niedrigem pH-Wert von 2 bis 6, insbesondere für Produkte zur Haar- und Hautreinigung und zur Körperpflege.

Die Verwendung von sauren Additiven und deren Salzen macht es teilweise notwendig, den pH-Wert der kosmetischen oder dermatologischen Zusammensetzungen A in einen deutlich sauren Bereich einzustellen.

In einer weiteren bevorzugten Ausführungsform enthalten die Zusammensetzungen A ein oder mehrere Hydroxysäuren, besonders bevorzugt ein oder mehrere Substanzen ausgewählt aus alpha- und beta-Hydroxysäuren.

An Hydroxysäuren können die Zusammensetzungen A vorzugsweise Milchsäure, Glykolsäure, Salicylsäure und alkylierte Salicylsäuren oder Zitronensäure enthalten. Weiterhin können Zusammensetzungen A weitere saure Komponenten enthalten. Als Wirkstoff in Betracht kommen Weinsäure, Mandelsäure, Caffeic acid, Brenztraubensäure, Oligooxa Mono- und Dicarbonsäuren, Fumarsäure, Retinoesäure, Sulfonsäuren, Benzoesäure, Kojisäure, Fruchtsäure, Äpfelsäure, Gluconsäure, Pyruvic Acid, Galacturonic Acid, Ribonic Acid, und all deren Derivate, Polyglykoldisäuren in freier oder teilweiser neutralisierter Form, Vitamin C (Ascorbinsäure), Vitamin C-Derivate, Dihydroxyaceton oder Skin-whitening Actives wie Arbutin oder Glycyrrhetinsäure und deren Salze. Der Gehalt an einer oder mehreren dieser soeben genannten Substanzen, bezogen auf die gesamte Zusammensetzung A, ist vorzugsweise von 0,1 bis 20,0 Gew.-%, besonders bevorzugt von 0,2 bis 10,0 Gew.-% und insbesondere bevorzugt von 0,5 bis 5,0 Gew.-%.

In einer weiteren bevorzugten Ausführungsform enthalten die Zusammensetzungen A daher ein oder mehrere Substanzen ausgewählt aus Vitamin C und Vitamin C-Derivaten, wobei die Vitamin C-Derivate vorzugsweise ausgewählt sind aus Natriumascorbylphosphat, Magnesiumascorbylphoshat und

Magnesiumascorbylglucosid.

In einer weiteren bevorzugten Ausführungsform enthalten die Zusammensetzungen A eine oder mehrere Substanzen ausgewählt aus Benzoesäure, Sorbinsäure, Salicylsäure, Milchsäure und Paramethoxybenzoesäure. Dadurch, dass die Polymere A auch im sauren pH-Bereich verdicken und eine Fließgrenze aufbauen, kann man mit den oben genannten organischen Säuren als Konservierer arbeiten.

Zu den Polymeren A können als zusätzliche Stabilisatoren Metallsalze von Fettsäuren, wie z. B. Magnesium-, Aluminium- und/oder Zinkstearat eingesetzt werden, bevorzugt in Mengen von 0,1 bis 10,0 Gew.-%, vorzugsweise von 0,5 bis 8,0 Gew.-% und besonders bevorzugt von 1,0 bis 5,0 Gew.-%, bezogen auf die fertigen Zusammensetzungen A.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guar-hydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxy-propyldiethylentriamin; Polyaminopolyamid und kationische Chitinderivate, wie beispielsweise Chitosan.

Die Zusammensetzungen A können einen oder mehrere der oben genannten kationischen Polymere in Mengen von 0,1 bis 5,0 Gew.-%, vorzugsweise von 0,2 bis 3,0 Gew.-% und besonders bevorzugt von 0,5 bis 2,0 Gew.-%, bezogen auf die fertigen Zusammensetzungen A, enthalten.

Des Weiteren können die Zusammensetzungen A Filmbildner enthalten, die je nach Anwendungszweck ausgewählt sind aus Salzen der Phenylbenzimidazolsulfonsäure, wasserlöslichen Polyurethanen, beispielsweise C₁₀ Polycarbamylpolyglycerylester, Polyvinylalkohol, Polyvinylpyrrolidoncopolymeren, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich.

Die Zusammensetzungen A können einen oder mehrere Filmbildner in Mengen von 0,1 bis 10,0 Gew.-%, vorzugsweise von 0,2 bis 5,0 Gew.-% und besonders bevorzugt von 0,5 bis 3,0 Gew.-%, bezogen auf die fertigen Zusammensetzungen A, enthalten.

Die gewünschte Viskosität der Zusammensetzungen A kann durch Zugabe von weiteren Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

In einer weiteren bevorzugten Ausführungsform enthalten die Zusammensetzungen A 0,01 bis 20,0 Gew.-%, besonders bevorzugt 0,1 bis 10,0 Gew.-%, insbesondere bevorzugt 0,2 bis 3,0 Gew.-% und ganz besonders bevorzugt 0,4 bis 2,0 Gew.-% an Verdickern bzw. Geliermitteln, bezogen auf die fertigen Zusammemnsetzungen A.

Als Überfettungsmittel oder Rückfetter können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden, bezogen auf die fertigen Zusammensetzungen A.

An antimikrobiellen Wirkstoffen können Cetyltrimethylammoniumchlorid, Cetylpyridiniumchlorid, Benzethoniumchlorid, Düsobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox^{®}, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol 1,2-Octandiol, 2 Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz kommen.

Die Zusammensetzungen A enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zusammensetzungen A.

Die Zusammensetzungen A können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol, Allantoin, Phytantriol, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die Zusammensetzungen A können biogene Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zusammensetzungen A, enthalten.

Die Zusammensetzungen A können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.-% und insbesondere bevorzugt in Mengen von 0,1 bis 10,0 Gew.-% enthalten..

Als deodorierende Stoffe bevorzugt sind Allantoin und Bisabolol. Diese werden vorzugsweise in Mengen von 0,0001 bis 10,0 Gew.-% eingesetzt.

In einer weiteren bevorzugten Ausführungsform enthalten die Zusammensetzungen A ein oder mehrere Substanzen ausgewählt aus anorganischen und organischen UV Filtern und liegen besonders bevorzugt in der Form einer Sonnenschutzzusammensetzung vor.

Die Zusammensetzungen A können als Pigmente/Mikropigmente sowie als anorganische Sonnenschutzfilter bzw. UV-Filter mikrofeines Titandioxid, Glimmer-Titanoxid, Eisenoxide, Glimmer-Eisenoxid, Zinkoxid, Siliciumoxide, Ultramarinblau oder Chromoxide enthalten. Die organischen Sonnenschutzfilter bzw. UV-Filter sind vorzugsweise ausgewählt aus 4-Aminobenzoesäure, 3 (4'-Trimethylammonium)-benzyliden-boran-2-on-methylsulfat, Camphor Benzalkonium Methosulfat, 3,3,5 Trimethyl-cyclohexylsalicylat, 2-Hydroxy-4-methoxybenzophenon, 2 Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäure-isoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)diimino]bis-(benzoesäure-2-ethylhexylester), Benzophenon-3, Benzophenon-4 (Säure), 3 (4' Methylbenzyliden)-D,L-Campher, 3-Benzyliden-Campher, Salicylsäure-2-ethylhexylester, 4 Dimethylaminobenzoesäure-2-ethylhexylester, Hydroxy-4-methoxy-benzophenon-5-sulfonsäure (Sulfisobenzonum) und das Natriumsalz, 4 Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2 2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphorsulfonsäure, Octocrylen, Polyacrylamidomethyl-Benzyliden-Camphor, 2 Ethylhexyl salicylat (Octyl Salicylat), 4 Dimethyl-aminobenzoesäureethyl-2-hexylester (octyl dimethyl PABA), PEG-25 PABA, 2 Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4 tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzimidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2 Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Die Menge der vorgenannten Sonnenschutzfilter (eine oder mehrere Verbindungen) in den Zusammensetzungen A beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der fertigen Zusammensetzung A. Die Zusammensetzungen A können einen oder mehrere Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L Carnosin und deren Derivaten (z. B. Anserin), Carotinoiden, Carotinen (z. B. Carotin, -Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. -Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den Zusammensetzungen A beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der Zusammensetzung A.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.-% und besonders bevorzugt von 0,5 bis 5,0 Gew.-%, bezogen auf die fertigen Zusammensetzungen A.

Zusätzlich können die Zusammensetzungen A organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, 1-Butanol, 2-Butanol, 2-Methylpropan-2-ol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1 Methoxy-2-propanol.

Die Zusammensetzungen A können ein oder mehrere Substanzen ausgewählt aus Farbmitteln, z.B. Farbstoffe und/oder Pigmente enthalten. Die in den Zusammensetzungen A enthaltenen Farbstoffe und/oder Pigmente, sowohl organische als auch anorganische Farbstoffe und Pigmente, sind aus der entsprechenden Positivliste der Kosmetikverordnung bzw. der EG-Liste kosmetischer Färbemittel ausgewählt.

**Chemische oder sonstige Bezeichnung CIN Farbe**

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1 "-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z. B. Paprikaextrakte, -Carotin und Cochenille.

Vorteilhaft eingesetzt werden auch Perlglanzpigmente, z. B. Fischsilber (Guanin/ Hypoxanthin-Mischkristalle aus Fischschuppen) und Perlmutt (vermahlene Muschelschalen), monokristalline Perlglanzpigmente wie z. B. Bismuthoxychlorid (BiOCl), Schicht-Substrat Pigmente, z. B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂O₃, TiO₂/Cr₂O3, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z.B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCl), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2 bis 50 µm, 5 bis 25 µm m, 5 bis 40 µm, 5 bis 60 µm, 5 bis 95 µm, 5 bis 100 µm, 10 bis 60 µm, 10 bis 100 µm, 10 bis 125 µm, 20 bis 100 µm, 20 bis 150 µm, sowie < 15 µm. Eine breitere Teilchengrößenverteilung z.B. von 20 bis 150 µm, ruft glitzernde Effekte hervor, während eine engere Teilchengrößenverteilung von < 15 µm für eine gleichmäßige seidige Erscheinung sorgt.

Die Zusammensetzungen A enthalten Effektpigmente vorzugsweise in Mengen von 0,1 bis 20,0 Gew.-%, besonders bevorzugt von 0,5 bis 10,0 Gew.-% und insbesondere bevorzugt von 1,0 bis 5,0 Gew.-%, jeweils bezogen auf das Gesamtgewicht der Zusammensetzungen.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykoldistearate mit durchschnittlich 3 Glykoleinheiten.

Sofern die Zusammensetzungen A perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zusammensetzungen enthalten.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Trübungsmittel können Polymerdispersionen insbesondere Polyacrylatderivat-, Polyacrylamidderivat-, Poly(acrylatderivat-co-acrylamidderivat)-Dispersionen, Poly(styrolderivate-co-acrylatderivat)-Dispersionen, gesättigte und ungesätigte Fettalkohole verwendet werden.

An Silikonen können die zuvor unter den Silikonölen bzw. -wachsen genannten Substanzen verwendet werden.

Als Säuren oder Laugen zur pH-Wert Einstellung können vorzugsweise Mineralsäuren, insbesondere HCl, anorganische Basen, insbesondere NaOH oder KOH, und organische Säuren, insbesondere Zitronensäure, verwendet werden.

Die Zusammensetzungen A haben pH-Werte von vorzugsweise 2 bis 14, besonders bevorzugt von 2 bis 10 und insbesondere bevorzugt von 2,5 bis 9.

In einer weiteren bevorzugten Ausführung liegen die Zusammensetzungen A in der Form von Duschgelen oder Shampoos vor.

Die Polymere A sind in vorteilhafter Weise als Verdicker, Konsistenzgeber, Emulgator, Sensorikadditiv, Solubilisator, Dispergator, Gleitmittel, Haftmittel, Stabilisator oder Fließgrenzenbildner geeignet.

Eine weitere bevorzugte Ausführung, ist die Verwendung eines oder mehrerer der Polymere A als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, außerordentlich bevorzugt in kosmetischen, dermatologischen oder pharmazeutischen Zusammensetzungen A.

Die Polymere A eignen sich besonders bevorzugt als als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, in flüssigen Zusammensetzungen, vorzugsweise in flüssigen kosmetischen, dermatologischen oder pharmazeutischen Zusammensetzungen, mit einem hohen Anteil an einem oder mehreren Tensiden.

Eine besonders bevorzugte Ausführungsform ist daher die Verwendung eines oder mehrerer der Polymere A als Verdicker, Konsistenzgeber, Emulgator, Sensorikadditiv, Solubilisator, Dispergator, Gleitmittel, Haftmittel, Stabilisator oder Fließgrenzenbildner, vorzugsweise als Verdicker, Konsistenzgeber oder Fließgrenzenbildner, besonders bevorzugt als Verdicker oder Fließgrenzenbildner und insbesondere bevorzugt als Fließgrenzenbildner, in flüssigen Zusammensetzungen A mit einem Tensidanteil von 0,01 bis 50,0 Gew.-%, vorzugsweise von 1,0 bis 30,0 Gew. %, besonders bevorzugt von 5,0 bis 20,0 Gew.-% und insbesondere bevorzugt von 5,0 bis 15,0 Gew.-%, außerordentlich bevorzugt in kosmetischen, dermatologischen oder pharmazeutischen Zusammensetzungen A.

In einer weiteren besonders bevorzugten Ausführungsform werden die Polymere A als Verdicker oder Fließgrenzenbildner, vorzugsweise als Fließgrenzenbildner, zur Stabilisierung von anorganischen oder organischen Partikeln, von Öltröpfchen oder von Gasblasen verwendet, besonders bevorzugt in kosmetischen, dermatologischen oder pharmazeutischen Zusammensetzungen A.

In einer weiteren besonders bevorzugten Ausführungsform werden die Polymere A als Verdicker oder Fließgrenzenbildner, vorzugsweise als Fließgrenzenbildner, zur Stabilisierung von anorganischen oder organischen Partikeln, von Öltröpfchen oder von Gasblasen in Zusammensetzungen auf wässriger, wässrig- alkoholischer, wässriger tensidhaltiger oder wässrig-alkoholischer tensidhaltiger Basis verwendet, vorzugsweise in entsprechenden kosmetischen, dermatologischen oder pharmazeutischen Zusammensetzungen A.

In einer weiteren besonders bevorzugten Ausführungsform werden die Polymere A als Verdicker oder Fließgrenzenbildner, vorzugsweise als Fließgrenzenbildner, in flüssigen Zusammensetzungen enthaltend ein oder mehrere Tenside zur Stabilisierung von anorganischen oder organischen Partikeln, von Öltröpfchen oder von Gasblasen verwendet, vorzugsweise in flüssigen kosmetischen, dermatologischen oder pharmazeutischen Zusammensetzungen A.

In einer insbesondere bevorzugten Ausführungsform werden die Polymere A als Verdicker oder Fließgrenzenbildner, vorzugsweise als Fließgrenzenbildner, zur Stabilisierung von anorganischen oder organischen Partikeln, von Öltröpfchen oder von Gasblasen in Zusammensetzungen A mit hohen Tensidkonzentrationen, vorzugsweise von 5,0 bis 20,0 Gew. % und besonders bevorzugt von 5,0 bis 15,0 Gew.-%, verwendet. Bei den Zusammensetzungen A handelt es sich vorzugsweise um kosmetische, dermatologische oder pharmazeutische Zusammensetzungen A. Derartige Zusammensetzungen A liegen vorzugsweise als klare Lösungen vor.

In einer weiteren bevorzugten Ausführungsform werden die Polymere A zur Stabilisierung von Emulsionen, vorzugsweise von salzhaltigen Emulsionen, besonders bevorzugt von salzhaltigen kosmetischen, dermatologischen oder pharmazeutischen Emusionen, verwendet.

Die Polymere A sind säurestabil und eignen sich in vorteilhafter Weise zur Verwendung in kosmetischen, dermatologischen oder pharmazeutischen Zusammensetzungen mit niedrigem pH-Wert, insbesondere für die Pflege und Behandlung der Körper- oder Gesichtshaut.

Weiterer Gegenstand der vorliegenden Erfindung ist daher die Verwedung eines oder mehrerer Polymere A zur Pflege und Behandlung der Körper- oder Gesichtshaut, vorzugsweise in kosmetischen, dermatologischen oder pharmazeutischen Zusammensetzungen A, besonders bevorzugt in sauren kosmetischen, dermatologischen oder pharmazeutischen Zusammensetzungen.

Vorzugsweise finden die Verwendungen der Polymere A in kosmetischen, pharmazeutischen oder dermatologischen Zusammensetzungen A statt.

Weiterhin vorteilhaft ist, dass die Polymere A auch ohne Mitverwendung eines zusätzlichen Fließgrenzebildners und/oder ohne Mitverwendung eines zusätzlichen Verdickers in den Zusammensetzungen A, vorzugsweise in kosmetischen, dermatologischen oder pharmazeutischen Zusammensetzungen A, eingesetzt werden können. Die Mitverwendung von zusätzlichen Fließgrenzebildnern und/oder Verdickern ist daher nicht zwingend, aber möglich. Eine Kombination mit anderen bekannten Fließgrenzenbildnern und/oder Verdickern kann zur Einstellung spezieller kosmetischer Profile und zur Ausnutzung synergistischer Effekte wünschenswert sein.

Weiterhin vorteilhaft ist, dass die Polymere A auch ohne Mitverwendung eines zusätzlichen Fließgrenzebildners und/oder Verdickers in wässrigen, wässrig-alkoholischen, wässrigen tensidhaltigen, wässrig-alkoholischen tensidhaltigen Zusammensetzungen, vorzugsweise in entsprechenden kosmetischen, dermatologischen oder pharmazeutischen Zusammensetzungen A, eingesetzt werden können.

### Beispiele:

### A) Verfahren:

In den nachfolgenden Beispielen des erfindungsgemäßen Verfahrens, dargelegt in den Verfahrensbeispielen 1 bis 12, werden thypische erfindungsgemäße Verfahren zur Herstellung der Polymere A näher erläutern, ohne diese darauf einzuschränken.

In den Beispielen wurde das verwendete polare Lösungsmittel variiert, mit deren Hilfe die Polymere C hergestellt werden können. Mit Hilfe der Verfahrensbeispiele 1 bis 20 wurden weitere erfindungsgemäße Polymere C durch die Variation der Monomere und Variation der Komponente e) herstellt. Diese Polymere C und das für die Synthese verwendete Verfahrensbeispielen werden in der Tabelle 1a) bis 1b) zusammengefasst.

### Verfahrensbeispiel 1:

In einem 2L Liter Quickfitkolben mit Ankerrührer, Rückflusskühler mit Abgaswäscher, kombiniertes Thermometer/pH-Meter und einem Gaseinleitungsrohr werden 20g wasserfreies 2-Methylpropan-2-ol und 368g Dimethylketon werden mit 12g destillierten Wasser versetzt. Das Reaktionsgefäß befindet sich in einem Heizbadthermostaten.Dieses Reaktionsgefäß wird mit Stickstoffgas überschichtet und im leichten Stickstoffgegenstrom 130g Acryloyldimethyltaurat und 53g Natriumhydrogencarbonat eingetragen. Das Acryloyldimethyltaurat Natrium Salz löst sich im 2-Methylpropan-2-ol /Dimethylketon / Wasser Gemisch nicht vollständig auf und liegt teilweise als Feststoffdispersion vor. Das Reaktionsgefäß wird mit Stickstoff überschichtet und es werden 1,8g Trimethylolpropantriacrylat werden eingetragen. Nach Eintrag des Trimethylolpropantriacrylat wird der pH-Wert kontrolliert und gegebenfalls durch Zusatz von Natriumhydrogencarbonat in den Bereich pH 7 bis 8 korrigiert. Es wird mindestens 1 Stunde ein konstanter Stickstoffstrom durch die Lösung geleitet. Nach dieser Inertisierungszeit wird der Restsauerstoff durch eine Sauerstoffelektrode überprüft. Sollte der gemessene Wert an Restsauerstoff in der flüssigen Phase den Wert 1ppm übersteigen, muss erneut inertisiert werden, bis dieser Wert erreicht wird. Danach werden der Reaktionskessel auf 60°C erwärmt im leichten Stickstoffstrom 2,0g Dilaurylperoxid zugegeben. Die Initiierung der Polymerisation wird durch einen Anstrieg der Innentemperatur erkennbar. Nach der Initiierung wird das Einleiten von Stickstoffgas beendet. Ungefähr 5-15 Minuten nach Einsetzen der Polymerisationsreaktion ist das Temperaturmaximum überschritten und die Temperatur im Reaktionsgefäß wird durch das Heizbad bis zum Siedepunkt des 2-Methylpropan-2-ol:Diemethylketon Wasser Gemisches erhöht. Unter leichten Rückfluss wird die nun viskose Masse zwei Stunden nachgerührt. Das Reaktionsprodukt, welches als Viskose Suspension von Polymer im 2-Methylpropan-2-ol:Diemethylketon Wasser Gemisch vorliegt, wird durch abfiltrieren und anschließender Trocknung im Vakuumtrockenschrank abgetrennt.

### Verfahrensbeispiel 2:

In einem 2L Liter Quickfitkolben mit Ankerrührer, Rückflusskühler mit Abgaswäscher, kombiniertes Thermometer/pH-Meter und einem Gaseinleitungsrohr werden 368g wasserfreies 2-Methylpropan-2-ol und 14g Dimethylketon werden mit 18g destillierten Wasser versetzt. Das Reaktionsgefäß befindet sich in einem Heizbadthermostaten. Die weiteren Schritte des Polymerisationsverfahrens 2 werden analog zu Polymerisationsverfahren 1 durchgeführt. Die Änderungen der Monomerzusammensetzungen sind in Tabelle 1 genau aufgeführt.

### Verfahrensbeispiel 3:

In einem 2L Liter Quickfitkolben mit Ankerrührer, Rückflusskühler mit Abgaswäscher, kombiniertes Thermometer/pH-Meter und einem Gaseinleitungsrohr werden 195g wasserfreies 2-Methylpropan-2-ol und 195g Dimethylketon werden mit 10g destillierten Wasser versetzt. Das Reaktionsgefäß befindet sich in einem Heizbadthermostaten. Die weiteren Schritte des Polymerisationsverfahrens 3 werden analog zu Polymerisationsverfahren 1 durchgeführt. Die Änderungen der Monomerzusammensetzungen sind in Tabelle 1 genau aufgeführt.

### Verfahrensbeispiel 4:

In einem 2L Liter Quickfitkolben mit Ankerrührer, Rückflusskühler mit Abgaswäscher, kombiniertes Thermometer/pH-Meter und einem Gaseinleitungsrohr werden 300g wasserfreies 2-Methylpropan-2-ol und 86g Dimethylketon werden mit 14g destillierten Wasser versetzt. Das Reaktionsgefäß befindet sich in einem Heizbadthermostaten. Die weiteren Schritte des Polymerisationsverfahrens 4 werden analog zu Polymerisationsverfahren 1 durchgeführt. Die Änderungen der Monomerzusammensetzungen sind in Tabelle 1 genau aufgeführt.

### Verfahrensbeispiel 5:

In einem 2L Liter Quickfitkolben mit Ankerrührer, Rückflusskühler mit Abgaswäscher, kombiniertes Thermometer/pH-Meter und einem Gaseinleitungsrohr werden 90g wasserfreies 2-Methylpropan-2-ol und 300g Dimethylketon werden mit 10g destillierten Wasser versetzt. Das Reaktionsgefäß befindet sich in einem Heizbadthermostaten. Die weiteren Schritte des Polymerisationsverfahrens 5 werden analog zu Polymerisationsverfahren 1 durchgeführt. Die Änderungen der Monomerzusammensetzungen sind in Tabelle 1 genau aufgeführt.

### Verfahrensbeispiel 6:

In einem 2L Liter Quickfitkolben mit Ankerrührer, Rückflusskühler mit Abgaswäscher, kombiniertes Thermometer/pH-Meter und einem Gaseinleitungsrohr werden und 388g Dimethylketon werden mit 12g destillierten Wasser versetzt. Das Reaktionsgefäß befindet sich in einem Heizbadthermostaten. Die weiteren Schritte des Polymerisationsverfahrens 6 werden analog zu Polymerisationsverfahren 1 durchgeführt. Die Änderungen der Monomerzusammensetzungen sind in Tabelle 1 genau aufgeführt.

### Verfahrensbeispiel 7:

In einem 2L Liter Quickfitkolben mit Ankerrührer, Rückflusskühler mit Abgaswäscher, kombiniertes Thermometer/pH-Meter und einem Gaseinleitungsrohr werden und 386g Dimethylketon werden mit 14g destillierten Wasser versetzt. Das Reaktionsgefäß befindet sich in einem Heizbadthermostaten. Die weiteren Schritte des Polymerisationsverfahrens 7 werden analog zu Polymerisationsverfahren 1 durchgeführt. Die Änderungen der Monomerzusammensetzungen sind in Tabelle 1 genau aufgeführt.

### Verfahrensbeispiel 8:

In einem 2L Liter Quickfitkolben mit Ankerrührer, Rückflusskühler mit Abgaswäscher, kombiniertes Thermometer/pH-Meter und einem Gaseinleitungsrohr werden und 390g Dimethylketon werden mit 10g destillierten Wasser versetzt. Das Reaktionsgefäß befindet sich in einem Heizbadthermostaten. Die weiteren Schritte des Polymerisationsverfahrens 8 werden analog zu Polymerisationsverfahren 1 durchgeführt. Die Änderungen der Monomerzusammensetzungen sind in Tabelle 1 genau aufgeführt.

### Verfahrensbeispiel 9:

In einem 2L Liter Quickfitkolben mit Ankerrührer, Rückflusskühler mit Abgaswäscher, kombiniertes Thermometer/pH-Meter und einem Gaseinleitungsrohr werden und 384g Tetrahydrofuran werden mit 16g destillierten Wasser versetzt. Das Reaktionsgefäß befindet sich in einem Heizbadthermostaten. Die weiteren Schritte des Polymerisationsverfahrens 9 werden analog zu Polymerisationsverfahren 1 durchgeführt. Die Änderungen der Monomerzusammensetzungen sind in Tabelle 1 genau aufgeführt.

### Verfahrensbeispiel 10:

In einem 2L Liter Quickfitkolben mit Ankerrührer, Rückflusskühler mit Abgaswäscher, kombiniertes Thermometer/pH-Meter und einem Gaseinleitungsrohr werden und 390g Tetrahydrofuran werden mit 10g destillierten Wasser versetzt. Das Reaktionsgefäß befindet sich in einem Heizbadthermostaten. Die weiteren Schritte des Polymerisationsverfahrens 10 werden analog zu Polymerisationsverfahren 1 durchgeführt. Die Änderungen der Monomerzusammensetzungen sind in Tabelle 1 genau aufgeführt.

### Verfahrensbeispiel 11:

In einem 2L Liter Quickfitkolben mit Ankerrührer, Rückflusskühler mit Abgaswäscher, kombiniertes Thermometer/pH-Meter und einem Gaseinleitungsrohr werden 300g wasserfreies 2-Methylpropan-2-ol und 86g 2-Methyltetrahydrofuran werden mit 14g destillierten Wasser versetzt. Das Reaktionsgefäß befindet sich in einem Heizbadthermostaten. Die weiteren Schritte des Polymerisationsverfahrens 11 werden analog zu Polymerisationsverfahren 1 durchgeführt. Die Änderungen der Monomerzusammensetzungen sind in Tabelle 1 genau aufgeführt.

### Verfahrensbeispiel 12:

In einem 2L Liter Quickfitkolben mit Ankerrührer, Rückflusskühler mit Abgaswäscher, kombiniertes Thermometer/pH-Meter und einem Gaseinleitungsrohr werden 90g wasserfreies 2-Methylpropan-2-ol und 300g 2-Methyltetrahydrofuran werden mit 10g destillierten Wasser versetzt. Das Reaktionsgefäß befindet sich in einem Heizbadthermostaten. Die weiteren Schritte des Polymerisationsverfahrens 12 werden analog zu Polymerisationsverfahren 1 durchgeführt. Die Änderungen der Monomerzusammensetzungen sind in Tabelle 1 genau aufgeführt.

Die Polymere A die nach den erfindungsgemäßen Verfahrensbeispielen 1 bis 12 hergestellt wurden, sind in der nachfolgenden Tabelle 1a bis b aufgeführt. Durchgeführte Änderungen wie beispielsweise der Einsatz und die Einsatzmenge einer anderen Base zur Neutralisation des Acryloyldimethyltaurat oder der Einsatz und die Einsatzmenge eines anderen Initiators sind in der Tabelle 1a bis b dargelegt.

**Tabelle 1a: Beispiele für Polymere hergestellt nach den erfindungsgemäßen Polymerisationsverfahren 1 bis 12**

| Name | Verf. Bsp. | ACDMT | | Neutralisierungsreagenz | | Vernetzer | | | V-65 | |
|---|---|---|---|---|---|---|---|---|---|---|
| | | g | mol% | Name | /g | Name | g | mol% | Name | g |
| Polymer - 1 | 1 | 100,0 | 99,0 | NaHCO₃ | 53 | TMPTA | 1,45 | 1,00 | DLP | 1,30 |
| Polymer - 2 | 2 | 100,0 | 97,0 | KHCO₃ | 48 | TMPTA | 4,35 | 2,95 | DLP | 2,00 |
| Polymer - 3 | 3 | 100,0 | 99,0 | Na₂CO₃ | 25,5 | TMPTA | 2,17 | 1,50 | DLP | 1,30 |
| Polymer - 4 | 3 | 100,0 | 99,0 | NaHCO₃ | 53 | TMPTA | 1,45 | 1,00 | DLP | 1,30 |
| Polymer - 5 | 5 | 100,0 | 98,7 | NaHCO₃ | 53 | TMPTA | 1,81 | 1,25 | DLP | 2,00 |
| Polymer - 6 | 1 | 100,0 | 98,5 | NaHCO₃ | 53 | TMPMTA | 2,30 | 1,52 | DLP | 2,00 |
| Polymer - 7 | 2 | 100,0 | 97,1 | NaHCO₃ | 53 | TMPMTA | 4,50 | 2,94 | V-601 | 1,10 |
| Polymer - 8 | 3 | 100,0 | 99,0 | NaHCO₃ | 53 | TMPMTA | 1,50 | 1,00 | V-601 | 1,10 |
| Polymer - 9 | 4 | 100,0 | 98,0 | Na₂CO₃ | 25,5 | TMPMTA | 3,05 | 2,01 | V-601 | 1,10 |
| Polymer - 10 | 5 | 100,0 | 98,7 | NaHCO₃ | 53 | TMPMTA | 1,89 | 1,25 | V-601 | 1,10 |
| Polymer - 11 | 1 | 100,0 | 99,0 | NaHCO₃ | 53 | PEG 600 DMA | 2,80 | 1,01 | V-601 | 1,10 |
| Polymer -12 | 2 | 100,0 | 97,0 | LiHCO₃ | 32,6 | PEG 600 DMA | 8,50 | 3,00 | DLP | 2,00 |
| Polymer -13 | 3 | 100,0 | 98,5 | NaHCO₃ | 53 | PEG 600 DMA | 4,25 | 1,52 | DLP | 2,00 |
| Polymer - 14 | 4 | 100,0 | 98,0 | NaHCO₃ | 53 | PEG 600 DMA | 5,60 | 2,00 | DLP | 2,00 |
| Polymer - 15 | 5 | 100,0 | 98,8 | NaHCO₃ | 53 | PEG 600 DMA | 3,48 | 1,25 | DLP | 2,00 |
| Polymer - 16 | 1 | 100,0 | 99,0 | Na₂CO₃ | 25,5 | GPTA | 2,06 | 0,99 | DLP | 2,00 |
| Polymer - 17 | 2 | 100,0 | 97,0 | LiHCO₃ | 32,6 | GPTA | 6,30 | 2,96 | V-601 | 1,10 |
| Polymer - 18 | 3 | 100,0 | 98,5 | NaHCO₃ | 53 | GPTA | 3,15 | 1,50 | V-601 | 1,10 |
| Polymer - 19 | 4 | 100,0 | 98,0 | KHCO₃ | 48 | GPTA | 4,20 | 1,99 | V-601 | 1,10 |
| Polymer - 20 | 5 | 100,0 | 98,8 | Na₂CO₃ | 25,5 | GPTA | 2,60 | 1,24 | V-601 | 1,10 |
| Polymer - 21 | 1 | 100,0 | 99,0 | KHCO₃ | 48 | PEAS | 2,50 | 1,00 | V-601 | 1,10 |
| Polymer - 22 | 2 | 100,0 | 97,0 | NaHCO₃ | 53 | PEAS | 7,60 | 2,99 | V-601 | 1,10 |
| Polymer - 23 | 3 | 100,0 | 98,5 | NaHCO₃ | 53 | PEAS | 3,80 | 1,52 | V-601 | 1,10 |
| Polymer - 24 | 4 | 100,0 | 98,5 | NaHCO₃ | 53 | PEAS | 3,75 | 1,50 | V-601 | 1,10 |
| Polymer - 25 | 5 | 100,0 | 98,8 | Na₂CO₃ | 25,5 | PEAS | 3,10 | 1,24 | V-601 | 1,10 |
| Polymer - 26 | 6 | 100,0 | 98,7 | NaHCO₃ | 53 | TMPTA | 1,81 | 1,25 | DLP | 2,00 |
| Polymer - 27 | 6 | 100,0 | 99,0 | Na₂CO₃ | 25,5 | TMPTA | 1,45 | 1,00 | DLP | 2,00 |
| Polymer - 28 | 6 | 100,0 | 98,5 | NaHCO₃ | 53 | TMPTA | 2,17 | 1,50 | DLP | 2,00 |
| Polymer - 29 | 6 | 100,0 | 98,5 | LiHCO₃ | 32,6 | TMPMTA | 2,30 | 1,52 | V-601 | 1,10 |
| Polymer - 30 | 6 | 100,0 | 98,8 | NaHCO₃ | 53 | PEG 600 DMA | 3,48 | 1,25 | DLP | 2,00 |
| Polymer - 31 | 6 | 100,0 | 98,8 | NaHCO₃ | 53 | GPTA | 2,60 | 1,24 | V-601 | 1,10 |
| Polymer - 32 | 6 | 100,0 | 98,5 | NaHCO₃ | 53 | PEAS | 3,75 | 1,50 | V-601 | 1,10 |
| Polymer - 33 | 7 | 100,0 | 98,7 | NaHCO₃ | 53 | TMPTA | 1,81 | 1,25 | DLP | 2,00 |
| Polymer - 34 | 7 | 100,0 | 99,0 | Na₂CO₃ | 25,5 | TMPTA | 1,45 | 1,00 | DLP | 2,00 |
| Polymer - 35 | 7 | 100,0 | 98,5 | KHCO₃ | 48 | TMPTA | 2,17 | 1,50 | DLP | 2,00 |
| Polymer - 36 | 7 | 100,0 | 98,5 | NaHCO₃ | 53 | TMPMTA | 2,30 | 1,52 | V-601 | 1,10 |
| Polymer - 37 | 7 | 100,0 | 98,8 | Na₂CO₃ | 25,5 | PEG 600 DMA | 3,48 | 1,25 | DLP | 2,00 |
| Polymer - 38 | 7 | 100,0 | 98,8 | NaHCO₃ | 53 | GPTA | 2,60 | 1,24 | V-601 | 1,10 |
| Polymer - 39 | 7 | 100,0 | 98,5 | NaHCO₃ | 53 | PEAS | 3,75 | 1,50 | V-601 | 1,10 |
| Polymer - 40 | 8 | 100,0 | 98,7 | NaHCO₃ | 53 | TMPTA | 1,81 | 1,25 | DLP | 2,00 |
| Polymer - 41 | 8 | 100,0 | 99,0 | LiHCO₃ | 32,6 | TMPTA | 1,45 | 1,00 | DLP | 2,00 |
| Polymer - 42 | 8 | 100,0 | 98,5 | NaHCO₃ | 53 | TMPTA | 2,17 | 1,50 | DLP | 2,00 |
| Polymer - 43 | 8 | 100,0 | 98,5 | Na₂CO₃ | 25,5 | TMPMTA | 2,30 | 1,52 | V-601 | 1,10 |
| Polymer - 44 | 8 | 100,0 | 98,8 | NaHCO₃ | 53 | PEG 600 DMA | 3,48 | 1,25 | DLP | 2,00 |
| Polymer - 45 | 8 | 100,0 | 98,8 | NaHCO₃ | 53 | GPTA | 2,60 | 1,24 | V-601 | 1,10 |

| | | | | | | | | | | |
|---|---|---|---|---|---|---|---|---|---|---|
| ACDMT = Acryloyldimethyltaurat, TMPTA = Trimethylolpropantriacrylat, TMPTA = Trimethylolpropantriacrylat, TMPTMA = Trimethylolpropantrimethacrylat, GPTA = Glycerinpropoxylattriacrylat, PEAS = Pentaerythritoldiacrylatmonostearat, DLP = Dilaurylperoxid, V-601 = Dimethyl 2,2'-azobis(2-methylpropionat) | | | | | | | | | | |

**Tabelle 1b: Beispiele für Polymere hergestellt nach den erfindungsgemäßen Polymerisationsverfahren 1 bis 12**

| Name | Verf. Bsp. | ACDMT | | Neutralisierungsreagenz | | Vernetzer | | | V-65 | |
|---|---|---|---|---|---|---|---|---|---|---|
| | | g | mol% | Name | /g | Name | g | mol% | Name | g |
| Polymer - 46 | 8 | 100,0 | 98,5 | NaHCO₃ | 53 | PEAS | 3,75 | 1,50 | V-601 | 1,10 |
| Polymer - 47 | 9 | 100,0 | 98,7 | LiHCO₃ | 32,6 | TMPTA | 1,81 | 1,25 | DLP | 2,00 |
| Polymer - 48 | 9 | 100,0 | 99,0 | KHCO₃ | 48 | TMPTA | 1,45 | 1,00 | DLP | 2,00 |
| Polymer - 49 | 9 | 100,0 | 98,5 | Na₂CO₃ | 25,5 | TMPTA | 2,17 | 1,50 | DLP | 2,00 |
| Polymer - 50 | 9 | 100,0 | 98,5 | KHCO₃ | 48 | TMPMTA | 2,30 | 1,52 | V-601 | 1,10 |
| Polymer - 51 | 9 | 100,0 | 98,8 | KHCO₃ | 48 | PEG 600 DMA | 3,48 | 1,25 | DLP | 2,00 |
| Polymer - 52 | 9 | 100,0 | 98,8 | LiHCO₃ | 32,6 | GPTA | 2,60 | 1,24 | V-601 | 1,10 |
| Polymer - 53 | 9 | 100,0 | 98,5 | KHCO₃ | 48 | PEAS | 3,75 | 1,50 | V-601 | 1,10 |
| Polymer - 54 | 10 | 100,0 | 98,7 | Na₂CO₃ | 25,5 | TMPTA | 1,81 | 1,25 | DLP | 2,00 |
| Polymer - 55 | 10 | 100,0 | 99,0 | KHCO₃ | 48 | TMPTA | 1,45 | 1,00 | DLP | 2,00 |
| Polymer - 56 | 10 | 100,0 | 98,5 | NaHCO₃ | 53 | TMPTA | 2,17 | 1,50 | DLP | 2,00 |
| Polymer - 57 | 10 | 100,0 | 98,5 | KHCO₃ | 48 | TMPMTA | 2,30 | 1,52 | V-601 | 1,10 |
| Polymer - 58 | 10 | 100,0 | 98,8 | LiHCO₃ | 32,6 | PEG 600 DMA | 3,48 | 1,25 | DLP | 2,00 |
| Polymer - 59 | 10 | 100,0 | 98,8 | KHCO₃ | 48 | GPTA | 2,60 | 1,24 | V-601 | 1,10 |
| Polymer - 60 | 10 | 100,0 | 98,5 | Na₂CO₃ | 25,5 | PEAS | 3,75 | 1,50 | V-601 | 1,10 |
| Polymer - 61 | 11 | 100,0 | 98,7 | KHCO₃ | 48 | TMPTA | 1,81 | 1,25 | DLP | 2,00 |
| Polymer - 62 | 11 | 100,0 | 99,0 | NaHCO₃ | 53 | TMPTA | 1,45 | 1,00 | DLP | 2,00 |
| Polymer - 63 | 11 | 100,0 | 98,5 | KHCO₃ | 48 | TMPTA | 2,17 | 1,50 | DLP | 2,00 |
| Polymer - 64 | 11 | 100,0 | 98,5 | KHCO₃ | 48 | TMPMTA | 2,30 | 1,52 | V-601 | 1,10 |
| Polymer - 65 | 11 | 100,0 | 98,8 | Na₂CO₃ | 25,5 | PEG 600 DMA | 3,48 | 1,25 | DLP | 2,00 |
| Polymer - 66 | 11 | 100,0 | 98,8 | KHCO₃ | 48 | GPTA | 2,60 | 1,24 | V-601 | 1,10 |
| Polymer - 67 | 11 | 100,0 | 98,5 | LiHCO₃ | 32,6 | PEAS | 3,75 | 1,50 | V-601 | 1,10 |
| Polymer - 68 | 12 | 100,0 | 99,0 | KHCO₃ | 48 | TMPTA | 1,45 | 1,00 | DLP | 2,00 |
| Polymer - 69 | 12 | 100,0 | 98,5 | NaHCO₃ | 53 | TMPTA | 2,17 | 1,50 | DLP | 2,00 |
| Polymer - 70 | 12 | 100,0 | 98,5 | KHCO₃ | 48 | TMPMTA | 2,30 | 1,52 | V-601 | 1,10 |
| Polymer - 71 | 12 | 100,0 | 98,8 | Na₂CO₃ | 25,5 | PEG 600 DMA | 3,48 | 1,25 | DLP | 2,00 |
| Polymer - 72 | 12 | 100,0 | 98,8 | LiHCO₃ | 32,6 | GPTA | 2,60 | 1,24 | V-601 | 1,10 |
| Polymer - 73 | 12 | 100,0 | 98,5 | KHCO₃ | 48 | GPTA | 3,15 | 1,50 | V-601 | 1,10 |
| Polymer - 74 | 12 | 100,0 | 98,5 | NaHCO₃ | 53 | PEAS | 3,75 | 1,50 | V-601 | 1,10 |
| Polymer - 75 | 12 | 100,0 | 99,0 | KHCO₃ | 48 | PEAS | 2,50 | 1,00 | V-601 | 1,10 |
| Polymer - 76 | 1 | 100,0 | 98,8 | NaHCO₃ | 53 | GPTA | 2,60 | 1,24 | DLP | 2,00 |
| Polymer - 77 | 1 | 100,0 | 98,8 | Na₂CO₃ | 25,5 | GPTA | 2,60 | 1,24 | DLP | 2,00 |
| Polymer - 78 | 1 | 100,0 | 98,8 | KHCO₃ | 48 | GPTA | 2,60 | 1,24 | DLP | 2,00 |
| Polymer - 79 | 1 | 100,0 | 98,8 | K₂CO₃ | 33 | GPTA | 2,60 | 1,24 | DLP | 2,00 |
| Polymer - 80 | 1 | 100,0 | 98,7 | LiHCO₃ | 32,6 | GPTA | 2,60 | 1,24 | DLP | 2,00 |
| Polymer - 81 | 1 | 100,0 | 98,7 | Li₂CO₃ | 17,7 | GPTA | 2,60 | 1,24 | DLP | 2,00 |
| Polymer - 82 | 1 | 100,0 | 98,7 | NaHCO₃ | 53 | TMPMTA | 1,89 | 1,25 | DLP | 2,00 |
| Polymer - 83 | 1 | 100,0 | 98,7 | KHCO₃ | 48 | TMPMTA | 1,89 | 1,25 | DLP | 2,00 |
| Polymer - 84 | 1 | 100,0 | 98,7 | Li₂CO₃ | 17,7 | TMPMTA | 1,89 | 1,25 | DLP | 2,00 |
| Polymer - 85 | 1 | 100,0 | 98,8 | NaHCO₃ | 53 | PEG 600 DMA | 3,48 | 1,25 | V-601 | 1,10 |
| Polymer - 86 | 1 | 100,0 | 98,8 | KHCO₃ | 48 | PEG 600 DMA | 3,48 | 1,25 | V-601 | 1,10 |
| Polymer - 87 | 1 | 100,0 | 98,8 | Li₂CO₃ | 17,7 | PEG 600 DMA | 3,48 | 1,25 | V-601 | 1,10 |
| Polymer - 88 | 1 | 100,0 | 98,7 | NaHCO₃ | 53 | TMPTA | 1,81 | 1,25 | V-601 | 1,10 |
| Polymer - 89 | 1 | 100,0 | 98,7 | KHCO₃ | 48 | TMPTA | 1,81 | 1,25 | V-601 | 1,10 |
| Polymer - 90 | 1 | 100,0 | 98,7 | Li₂CO₃ | 17,7 | TMPTA | 1,81 | 1,25 | V-601 | 1,10 |

| | | | | | | | | | | |
|---|---|---|---|---|---|---|---|---|---|---|
| ACDMT = Acryloyldimethyltaurat, TMPTA = Trimethylolpropantriacrylat, TMPTA = Trimethylolpropantriacrylat, TMPTMA = Trimethylolpropantrimethacrylat, GPTA = Glycerinpropoxylattriacrylat, PEAS = Pentaerythritoldiacrylatmonostearat, DLP = Dilaurylperoxid, V-601 = Dimethyl 2,2'-azobis(2-methylpropionat) | | | | | | | | | | |

### Vergleichsbeispiel A1 (nicht erfindungsgemäß, hergestellt nach EP0816403B1 Ammonium-Salz, NH4⁺-Salz)

In einem 5,0L Liter Quickfitkolben mit Ankerrührer, Rückflusskühler mit Abgaswäscher, kombiniertes Thermometer/pH-Meter und einem Gaseinleitungsrohr für N₂ und NH₃ (gasförmig) werden 2000 g 2-Methylpropan-2-ol (97,5%-ig, Rest Wasser) vorgelegt. Das Reaktionsgefäß befindet sich in einem Heizbadthermostaten.

Dieses Reaktionsgefäß wird mit Stickstoffgas überschichtet und im leichten Stickstoffgegenstrom 340,0 g Acryloyldimethyltaurat eingetragen und unter straken Rühren dispergiert, wobei eine leichte Trübung des Lösungsmittels erhalten bleibt. Über einen Zeitraum von 30 Minuten leitet man nun 27-28g Ammoniak (gasförmig, etwa äquimolar mit der Menge an Acryloyldimethyltaurat in den überstehenden Gasraum ein, und rührt weitere 30Minuten bei Raumtemperatur nach bzw. bis sich ein pH-Wert von 6,0±0,5 eingestellt hat. Man gibt 32,0g TMPTA-Lösung (Trimethylolprpantriacrylat) 25%-ig in 2-Methylpropan-2-ol zu und erwärmt auf eine Temperatur von T=60°C , wobei die Reaktionsmischung durch gleichzeitiges Einleiten von Stickstoff inertisiert wird. Nach Erreichen der Temperatur von 60°C werden 4,0g DLP (Dilaurylperoxid) zugegeben. Die Reaktion springt unmittelabr nach der Zugabe des Initiators an, was an einen Anstieg der Temperatur und am Ausflocken des Polymerisates zu erkennen ist. Etwas 15 Minuten nach dem Einsetzen der Polymerisationsreaktion wird die Stickstoffzufuhr eingestellt. Ungefähr 30 Minuten nach Zugabe des Starters DLP erreicht die Temperatur ein Maximum (ca. 65-70°C). Weitere 30 Minuten nach Durchlaufen dieses Maximums wird zum Rückfluss erhitzt und unter diesen Bedingungen zwei Stunden nachgerührt. Der Inhalt des Reaktionsgefäßes nimmt im Verlauf der Reaktion eine breiartige Konsistenz an, ist aber noch gut rührbar. Anschließend wird auf Raumtemperatur abgekühlt und der Feststoff abgesaugt. Die Paste wird bei 60-70°C über 24h im Vakuumtrockenschrank getrocknet.

Man erhält: 390g (95,5 Gew % Feststoffgehalt) Vergleichspolymer A1

### Vergleichsbeispiel A2 (nicht erfindungsgemäß, hergestellt nach EP0816403B1 analog als Na⁺-Salz)

In einem 5,0L Liter Quickfitkolben mit Ankerrührer, Rückflusskühler mit Abgaswäscher, kombiniertes Thermometer/pH-Meter und einem Gaseinleitungsrohr für N₂ und NH₃ (gasförmig) werden 2000 g 2-Methylpropan-2-ol (97,5%-ig, Rest Wasser) vorgelegt. Das Reaktionsgefäß befindet sich in einem Heizbadthermostaten.

Dieses Reaktionsgefäß wird mit Stickstoffgas überschichtet und im leichten Stickstoffgegenstrom 340,0g Acryloyldimethyltaurat und 168g Natriumhydrogencarbonat eingetragen unter straken Rühren dispergiert, wobei eine leichte Trübung des Lösungsmittels erhalten bleibt. Man gibt 32,0g TMPTA-Lösung (Trimethylolprpantriacrylat) 25%-ig in 2-Methylpropan-2-ol zu und erwärmt auf eine Temperatur von T=60°C , wobei die Reaktionsmischung durch gleichzeitiges Einleiten von Stickstoff inertisiert wird. Nach Erreichen der Temperatur von 60°C werden 4,0g DLP (Dilaurylperoxid) zugegeben. Die Reaktion springt nach 5-10 Minuten nach der Zugabe des Initiators an, was an einen Anstieg der Temperatur und am Ausflocken des Polymerisates zu erkennen ist. Etwas 20-25 Minuten nach dem Einsetzen der Polymerisationsreaktion wird die Stickstoffzufuhr eingestellt. Ungefähr 30 Minuten nach Zugabe des Starters DLP erreicht die Temperatur ein Maximum (ca. 65-70°C). Weitere 30 Minuten nach Durchlaufen dieses Maximums wird zum Rückfluss erhitzt und unter diesen Bedingungen zwei Stunden nachgerührt. Der Inhalt des Reaktionsgefäßes nimmt im Verlauf der Reaktion eine breiartige Konsistenz an, ist aber noch gut rührbar. Anschließend wird auf Raumtemperatur abgekühlt und der Feststoff abgesaugt. Die Paste wird bei 60-70°C über 24h im Vakuumtrockenschrank getrocknet.

Man erhält: 405g (93,6 Gew % Feststoffgehalt) Vergleichspolymer A2

### Vergleichsbeispiel B1 (nicht erfindungsgemäß, hergestellt nach US2012095120)

In einen 2L Glasreaktor mit einer Innentemperatur von 20°C werden 344g Dimethylketon, 50,8g Acryloyldimethyltaurat, 20,8g NaHCO₃, 9,6g deionisiertes Wasser und 0,45g Trimethylolprpantriacrylat eingeführt. Der Reaktorinhalt wird gerührt und unter starken Stickstoffeinleitung für 1 h inertisiert. Das Reaktionsmedium wird auf 55°C erhitzt und dann werden 0,75g DLP (Dilaurylperoxid) zum Start der Polymerisation hinzugegeben. Die Reaktionsmischung wird auf Rückfluss erhitzt und für 2h gehalten. Nach dem Abkühlen auf Raumtemperatur wird das Reaktionsmedium filtriert und der Polymerrückstand unter Vakuum getrocknet.

### Man erhält 58,2g (93,8Gew% Feststoffgehalt) Vergleichspolymer B1

Viskositätsbestimmung der erfindungsgemäßen Polymere und der Vergleichsbeispiele A1, A2 und B1

Die Bestimmung der Viskosität der wässrigen Hydrogele erfolgte mit Hilfe eines Brookfield RVT Viskosimeters. In einem Becherglas werden 4g der zu untersuchenden Polymere und 396g destillierten Wasser eingewogen und unter rühren (200rpm) gelöst. Nach 12h werden die erhaltenenen Hydrogele in einem Wasserbad auf 25,0°C (±0,2°C) temperiert und mit Hilfe des Brookfield RVT Viskosimeters (25 °C, T-C Spindel bei 5 rpm) vermessen (Tabelle 3). Nach der Messung wurden die Gele optisch anhand der nachfolgeden Skala (Tabelle 2) bewertet. Ein Wert von 1 oder 2 entspricht einer sehr guten Beurteilung und einer deutlichen Verbessung des Stands der Technik.

**Tabelle 2: Bewerungsskala der Hydrogele**

| Wert | 1 | 2 | 3 | 4 |
|---|---|---|---|---|
| Bedeutung | Glasklar / Kristallklar, Transparent | Klar bis Leichte Trübung | Trüb | Sehr trüb teilweise nichtgelöste Bestandteile |

**Tabelle 3: Viskositätsbestimmung der erfindungsgemäßen Polymere und der Vergleichsbeispiele A1, A2 und B1**

| Polymer | Verfahren | Viskosität 1 Gew.-% in Wasser/mPa*s | Optische Beurteilung | Freisetzung von NH3 unter alkalischen Bedingungen |
|---|---|---|---|---|
| Polymer - 1 | 1 | 22700 | 2 | Nein |
| Polymer - 2 | 2 | 58400 | 2-3 | Nein |
| Polymer - 3 | 3 | 55100 | 1-2 | Nein |
| Polymer - 4 | 3 | 38200 | 1 | Nein |
| Polymer - 8 | 3 | 48600 | 2 | Nein |
| Polymer - 13 | 3 | 20500 | 1 | Nein |
| Polymer - 14 | 4 | 31400 | 1 | Nein |
| Polymer - 18 | 3 | 35700 | 1 | Nein |
| Polymer - 20 | 5 | 46800 | 1-2 | Nein |
| Polymer - 23 | 3 | 52300 | 2 | Nein |
| Polymer - 31 | 6 | 22000 | 2 | Nein |
| Vergleichsbeispiele nicht erfindungsgemäß | | | | |
| VGL-A1 (NH₄⁺-Salz) | A1 | 34400 | 1 | JA |
| VGL-A2 (Na⁺-Salz) | A2 | 92 | 4 | Nein |
| VGL-B1 (Na⁺-Salz) | B1 | 16000 | 3-4 | Nein |

| | | | | |
|---|---|---|---|---|
| VGL-A1 = Vergleichspolymer A1, VGL-A2 = Vergleichspolymer A2, VGL-B1 = Vergleichspolymer B1, | | | | |

Wie der Vergleich der erfindungsgemäßen Beispiele in Tabelle 3 mit den Vergleichsbeispielen A1, A2 und B1 zeigt, wird mit dem erfindungsgemäßen Verfahren, das ein Lösemittelgemisch nutzt, ein Produkt erhalten welches sich von Produkten unterscheidet, die nach dem Stand der Technik mit nur einem Lösemittel erhalten wurden. Die nach dem erfindungsgemäßen Verfahren erhaltenen Produkte zeigen eine deutlich höhere Viskosität, erreichen eine verbesserte Bewertung in der Klarheit der Hydrogele und es kommt zu keiner Freisetzung von Ammoniak unter alkalischen Bedingungen.

## Patentansprüche

1. Verfahren zur Herstellung von wasserlöslichen oder wasserquellbaren Polymeren enthaltend:
a) 95 - 99,99 Mol-% vorzugsweise 97 bis 99,9 Mol-% einer oder mehrerer wiederkehrender Struktureinheiten der Formel (1) 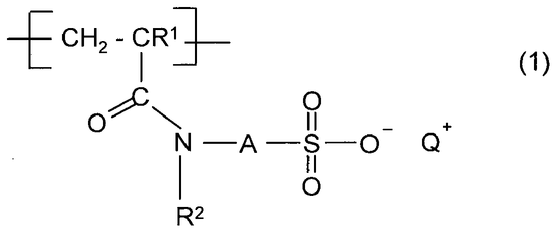 worin
R¹, R² unabhänig voneinander, Wasserstoff, Methyl oder Ethyl bedeuten,
A lineares oder verzweigtes C₁-C₁₂-Alkylen bedeutet, und
Q⁺ für H⁺, Li⁺, Na⁺, K⁺, ½ Ca⁺⁺, ½ Mg⁺⁺, ½ Zn⁺⁺, 1/3 Al⁺⁺⁺oder für Mischungen dieser Ionen steht, vorzugsweise für Na⁺ steht,
b) 0,01 bis 5, vorzugsweise 0,01 bis 3 Mol-% an einer oder mehreren voneinander unabhängigen wiederkehrenden vernetzenden Struktureinheiten, die aus einem oder mehreren Monomeren mit mindestens zwei olefinischen Doppelbindungen hervorgegangen sind,
**dadurch gekennzeichnet, dass** Monomere, aus denen sich die Struktureinheiten der Komponenten a) und b) ableiten, in einem polaren Lösungsmittel oder Lösungsmittelgemisch, in Fällung radikalisch polymerisiert werden, wobei in dem Fall das polare Lösungsmittel oder Lösungsmittelgemisch 2-Methyl-2-Propanol oder ein Keton enthält, ein zweites polares organisches Lösungsmittel enthalten sein muss, und gegebenenfalls die Monomere vor der Polymerisation oder das Polymer nach der Polymerisation mit einer Li⁺-, Na⁺-, K⁺-, Ca⁺⁺-, Mg⁺⁺-, Zn⁺⁺- oder Al⁺⁺⁺-enthaltenden Base vorzugsweise mit den entsprechenden Hydroxiden, Hydrogencarbonaten und Carbonaten, neutralisiert werden.

2. Verfahren nach Anspruch 1, **dadurch gekennzeichnet, dass** eine oder mehrere Struktureinheiten der Formel (1) abgeleitet sind von Monomeren aus der Gruppe bestehend aus Acryloyldimethyltaurat, Acryloyl-1,1-dimethyl-2methyltaurat, Acryloyltaurat, Acryloyl-N-methyltaurat.

3. Verfahren nach einem oder mehreren der Ansprüche 1 bis 2, **dadurch gekennzeichnet, dass** der Neutralisationsgrad der einen oder mehreren Struktureinheiten der Formel (1) von 50,0 bis 100 Mol-%, besonders bevorzugt von 80,0 bis 100 Mol-%, insbesondere bevorzugt von 90,0 bis 100 Mol-% und außerordentlich bevorzugt von 95,0 bis 100 Mol-% beträgt.

4. Verfahren nach einem oder mehreren der Ansprüche 1 bis 3, **dadurch gekennzeichnet, dass** eine oder mehrere Struktureinheiten der Komponente b) von Monomeren der allgemeinen Formel (2) abgeleitet sind: 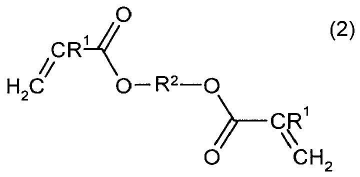 worin
R¹ Wasserstoff, Methyl oder Ethyl bedeutet und
R² eine lineare oder verzweigte Alkylengruppe mit 1 bis 6 Kohlenstoffatomen oder eine lineare oder verzweigte, einfach oder mehrfach ungesättigte Alkenylengruppe mit 2 bis 6 Kohlenstoffatomen ist.

5. Verfahren nach einem oder mehreren der Ansprüche 1 bis 4, **dadurch gekennzeichnet, dass** eine oder mehrere Struktureinheiten der Komponente b) von Struktureinheiten der allgemeinen Formel (3) abgeleitet sind: 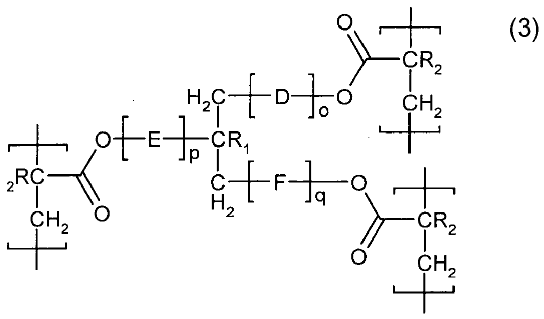 worin
R₁ eine lineare oder verzweigte Alkylengruppe mit 1 bis 6 Kohlenstoffatomen bedeutet und
R² jeweils unabhängig voneinander Wasserstoff, Methyl oder Ethyl bedeuten,
D, E und F jeweils unabhängig voneinander Methylenoxy (-CH₂-O- oder -O-CH₂-), Ethylenoxy (CH₂-CH₂-O- oder -O-CH₂-CH₂-), Popylenoxy (-CH(CH₃)-CH₂-O oder -O-CH₂-CH(CH₃)-), eine lineare oder verzweigte Alkylengruppe mit 1 bis 6 Kohlenstoffatomen, eine lineare oder verzweigte, einfach oder mehrfach ungesättigte Alkenylengruppe mit 2 bis 6 Kohlenstoffatomen, eine lineare Mono-Hydroxyalkylengruppe mit 2 bis 6 Kohlenstoffatomen oder eine lineare oder verzweigte Di-Hydroxyalkylengruppe mit 3 bis 6 Kohlenstoffatomen bedeuten, und
o, p und q jeweils unabhängig voneinander eine ganze Zahl von 0 bis 50, vorzugsweise 0 bis 25 und insbesondere bevorzugt von 0 bis 10 bedeutet.

6. Verfahren nach einem oder mehreren der Ansprüche 1 bis 5, **dadurch gekennzeichnet, dass** eine oder mehrere Struktureinheiten der Komponente b) von Monomeren aus der Gruppe bestehend aus Glycerinpropoxylattriacrylat (GPTA), Trimethylolpropantriacrylat (TMPTA), Hexandioldiacrylat (HDDA), Pentaerythritoldiacrylatmonostearat (PEAS), und Hexandioldimethacrylat (HDDMA) ausgewählt sind.

7. Verfahren nach einem oder mehreren der Ansprüche 1 bis 6, **dadurch gekennzeichnet, dass** Monomere, aus denen sich die Struktureinheiten der Komponenten a) und b) ableiten und gegebenenfalls die Monomere vor der Polymerisation oder das Polymer nach der Polymerisation mit einer Base aus der Gruppe bestehend aus Natriumhydrogencarbonat, Natriumcarbonat, Natriumhydroxid, Kaliumhydrogencarbonat, Kaliumcarbonat, Kaliumhydroxid, Lithiumhydrogencarbonat, Lithiumcarbonat, Lithiumhydroxid neutralisiert werden.

8. Verfahren nach einem oder mehreren der Ansprüche 1 bis 7, **dadurch gekennzeichnet, dass** das polare Lösungsmittel einen Siedepunkt von 60 bis 110 °C, vorzugsweise von 60 bis 85 °C, besonders bevorzugt von 70 bis 85 °C aufweist.

9. Verfahren nach einem oder mehreren der Ansprüche 1 bis 8, **dadurch gekennzeichnet, dass** das polare Lösungsmittel ein Lösungsmittelgemisch aus:
c) Wasser
und
d) einem oder mehreren weiteren polaren Lösungsmitteln ist.

10. Verfahren nach einem oder mehreren der Ansprüche 1 bis 9, **dadurch gekennzeichnet, dass** das polare Lösungsmittel ein Lösungsmittelgemisch ist, welches zwei oder mehr polare organische Lösungsmittel enthält, vorzugsweise dass das polare Lösungsmittel ein Lösungsmittelgemisch ist, welches ein oder mehrere Alkohole und ein oder mehrer Ketone enthält.

11. Verfahren nach einem oder mehreren der Ansprüche 9 bis 10, **dadurch gekennzeichnet, dass** die Komponente d) ein oder mehrere polare organische Lösungsmittel ausgewählt aus der Gruppe aus Methanol, Ethanol, 1-Propanol, 2-Propanol, 2-Methyl-2-Propanol, 1-Butanol, 2-Butanol, Dimethylketon, Diethylketon, Pentan-2-on, Butanon, Tetrahydropyran, Tetrahydrofuran, 2-Methyltetrahydrofuran, 1,3-Dioxan, 1,4-Dioxan enthält.

12. Verfahren nach einem oder mehreren der Ansprüche 1 bis 11, **dadurch gekennzeichnet, dass** das polare Lösungsmittel 0,5 bis 10 Gew.-% Wasser, 1 bis 98,5 Gew.-% 2-Methylpropan-2-ol und 1 bis 98,5 Gew.-% Dimethylketon enthält, vorzugsweise dass das polare Lösungsmittel 0,5 bis 7,5 Gew.-% Wasser, 5 bis 94,5 Gew.-% 2-Methylpropan-2-ol und 5 bis 94,5 Gew.-% Dimethylketon enthält, besonders bevorzugt dass das polare Lösungsmittel 1 bis 5 Gew.-% Wasser, 7,5 bis 91,5 Gew.-% 2-Methylpropan-2-ol und 7,5 bis 91,5 Gew.-% Dimethylketon enthält.

13. Verfahren nach einem oder mehreren der Ansprüche 1 bis 12, **dadurch gekennzeichnet, dass** das polare Lösungsmittel nach dem Polymerisationsprozess durch eine Filtration, vorzugsweise Druckfiltration, oder Destillation vom Produkt abgetrennt wird.

14. Wasserlösliches oder wasserquellbares Polymer erhältlich nach einem Verfahren gemäß einem oder mehreren der Ansprüche 1-13.

15. Kosmetische, dermatologische oder pharmazeutische Formulierung umfassend ein oder mehrere Wasserlösliche oder wasserquellbare Polymer, nach Anspuch 14.
